# EUROPEAN PATENT APPLICATION

(11) **EP 2 441 520 A1**
(43) Date of publication of application: **18.04.2012**
(21) Application number: 10187349.5
(22) Date of filing: 12.10.2010
(51) Int. Cl.: B01L 7/00, B01L 3/00, G01N 21/64, C12Q 1/68

(54) **Real-time amplification and micro-array based detection of nucleic acid targets in a flow chip assay**

(71) Applicant: Eppendorf AG, 22339 Hamburg (DE)
(72) Inventor: Alexandre, Isabelle, B-5340, HALTINNE (BE); Deroeck, Sven, B-1030, BELGIUM (BE); Remacle, Jose, B-5100, JAMBES (BE); Pluester, Wilhelm, B-3080, TERVUEREN (BE)
(74) Representative: den Hartog, Jeroen H.J.

(57) **Abstract**

The present method is related to a method for identification and/or quantification of at least one polynucleotide target compound present in a biological sample among possible other ones by its amplification in a cycling flow chip solution passing through different temperatures required for the amplification and its detection in real-time onto a micro-array of specific capture molecules.

## Description

### Field of the invention

The present invention relates to a process for conducting fast detection and/or quantification of target polynucleotide molecule(s). The present invention relates to a method for fast amplification, preferably by PCR, of multiple target polynucleotide molecules possibly present in a solution and quantitative real-time amplification of such targets combining micro-array and a flow through device. The method is well suited for diagnostic applications of complex sample in molecular biology.

The invention also provides a simple and non expensive device and instrument to conduct the method based on the fast detection and/or quantification of multiple targets by real-time follow up of the amplification.

### Description of the related art

Amplification of nucleic acid sequences is performed using a variety of amplification reactions, among them and most prominent the polymerase chain reaction (PCR). PCR is a method for amplification of specific nucleic acid sequences. When the amplification process is monitored in real-time, PCR is used for quantitative analysis. Real-time PCR normally uses fluorescent reporters in solution, such as intercalating dyes, TaqMan probes, Scorpion primers, molecular beacons. When several nucleic acid target sequences are to be analyzed in real time PCR, two approaches are proposed. The first one is to parallelize the reactions, i.e. to run each reaction in a separate compartment. The second approach is to multiplex the reactions, i.e. to run the reactions in the same compartment and to use different fluorochrome reporters for each reaction. This analysis is limited by the number of fluorochromes that are efficiently discriminated. The current state-of-the-art is normally 2 and sometimes 4 and rarely 6 reactions in the same solution.

The use of capture molecules to detect amplified polynucleotide during the PCR has been recently proposed (WO2006/053770) as a third alternative for multiplex real time PCR. The method is based on the use of the surface of an optical block on which capture molecules are fixed. They capture the amplified polynucleotides between the different PCR cycles so that it is possible to detect their appearance along the cycles and to draw a real-time curve for each target. The method uses a cartridge which is successively heated at different temperatures in order to make the 4 steps of the process: denaturation, annealing, elongation and hybridization. The thermal requirements of the PCR process and of the surface hybridization process are very different. The PCR amplification requires temperature cycling with the denaturation step being at a very high temperature (about 95°C) in order to be above the dsDNA melting temperature. On the other hand the surface hybridization needs to take place at an accurate temperature below the nucleic acid melting point, and for optimum performances diffusion limited local depletion of amplicons above the capture probe sites should be avoided. The method has been proposed with different methods of detection of the hybridized amplicons: confocal scanning (WO06053770), evanescence field (WO 2008/034896) and forbidden angle (WO2009/013220). This method is suggested to allow a detection of multiple target amplicons in about 3 hr or more.

There is a need for improvement of the real-time amplification method for multiple targets in order to obtain a completely automated, fast (e.g. less than 1 or two hours) and easy detection with the required sensitivity, reproducibility and quantification.

Single, or low number target PCR has been described in relatively fast cycling times. For example, Kopp et al 1998 (Science 280, 1046-1048), proposes a chip for flow-through PCR having a channel passing several times through 3 different temperature regions for denaturation, annealing and elongation. The channel has an entry for injecting the amplification solution and an outlet for recovery of the amplified product which is then analyzed by the standard methods such as electrophoresis on gel. Further, US 6,960,437 describes a microfluidic device having a rotary microfluidic channel and a plurality of temperature regions present at different locations along this rotary channel. The microfluidic channels are manufactured from elastomeric materials which deform when force is applied, but then return to their original shape when the force is removed. By cycling to the channel, the solution passed through the 3 amplification steps and the PCR is performed. The patent described the different parts and feature of a cycling device for obtaining a good amplification. Detection is performed in the solution using either TaqMan probe (example 3) or SYBR Green or after gel electrophoresis. The patent mentions the use of a micro-array which can be incorporated into the cycling channel so as to perform the detection of the amplified target. There is however neither mention nor indication of how to perform the detection on a micro-array in such a system.

The patent application EP-A-2138587 also proposed to amplify nucleic acid sequences by consecutive passages of the solution to 3 temperature zones and to perform detection on a micro-array having immobilized capture molecules. The methods and devices are characterized in that hybridization and detection are performed in a designated hybridization zone such that hybridization and detection can be adjusted independently from the amplification process. The invention provides decoupled optimization of these two processes. The preferred embodiment is a unidirectional non cycling flow through different parts of the channel for the different cycles having a dedicated hybridization zone for each cycle. The patent describes different configurations of the channel passing through different temperature zones. Preferably, a single scan is performed at the end of the PCR assay over multiple hybridization zones corresponding to a given cycle (frozen picture of spots with hybridized amplicons for a given cycle and a given hybridization time), in order to avoid the requirement of fast scanning at each cycle. One result is presented on this process of unidirectional flow device. The document is silent on how to perform homogeneous hybridization over the different discrete regions present on the micro-array surface during the amplification.

### Summary of the invention

The present method is related to a method for identification and/or quantification of at least one polynucleotide target compound present in a biological sample among possible other ones by its amplification in a cycling flow chip solution passing through different temperatures required for the amplification and its detection in real-time onto a micro-array of specific capture molecules.

As recited in claim 1, the present invention is related to a method for performing real-time amplification and detection of target polynucleotide molecule(s) present in a sample, comprising the steps of:
a) providing a flow chip device comprising:
   - a flow channel having a section comprised between 0.01 and 10 mm² and a volume V1, wherein the flow channel is configured such that a solution introduced into the flow channel is cycled through the flow channel,
   - a detection chamber in fluid communication with the flow channel, said detection chamber having an optically transparent solid support and a micro-array comprising more than 4 capture molecules being immobilized in localized areas of the surface of said transparent solid support, wherein said chamber has a height lower than 1 mm and a volume V2, and wherein the ratio V2/V1 is between 0.001 and 0.5,
   - at least 2 and preferably 3 different temperature regions at which temperature is regulated, each temperature region being located at a different location of the flow channel, wherein one temperature region comprises the detection chamber and has a temperature allowing the hybridization of the target polynucleotide molecules to the capture molecules,
b) introducing a solution having a volume V3 and containing target polynucleotide molecules into the flow channel and reagents for polynucleotide molecule amplification, wherein the ratio V3/(Vl+V2) is higher than 0.02 and lower than l,
c) submitting the solution to at least 5 amplification cycles to obtain labeled target polynucleotide molecules, wherein one amplification cycle is obtained by cycling the solution through the flow channel and the same detection chamber between the different temperature regions and wherein an amplification cycle is performed in less than 3 min,
d) measuring a fluorescent signal at different timings of the amplification from the hybridized target polynucleotide molecules in at least 3 and preferably in at least 5 different amplification cycles by detecting the fluorescence emitted from the localized areas of the surface having hybridized target polynucleotide measured after excitation of the fluorochrome by a light beam,
e) analyzing the signal values obtained from the localized area in order to detect and/or to quantify the target polynucleotide molecule(s) present in the sample.

The invention is also related to a device for performing such a method. The device is a flow-chip device for performing real-time amplification and detection of target polynucleotide molecule(s) present in a sample, comprising:
- a flow channel disposed within a substrate, said flow channel having a section comprised between 0.01 and 10 mm² and a volume V1, wherein the flow channel is configured such that a solution introduced into the flow channel is cycled through the flow channel,
- a detection chamber connected to the flow channel, said detection chamber having fixed upon one of its surface a micro-array comprising more than 4 capture molecules being immobilized in localized areas of said surface, wherein said chamber has a height lower than 1 mm and a volume V2, and wherein the ratio V2/V1 is between 0.001 and 0.5,

- at least 2 and preferably 3 different temperature regions at which temperature is regulated, each temperature region being located at a different location of the flow channel, wherein one temperature region comprises the detection chamber and has a temperature allowing the hybridization of the target polynucleotide molecules to the capture molecules,
- an inlet in fluid communication with the flow channel via which the solution is introduced into the flow channel,
wherein the capture molecules are immobilized on the surface (S1) of an optically transparent solid support having a refractive index higher than 1.3 and a thickness of at least 0.5 mm and preferably at least 3 mm, wherein said solid support has two surfaces (S2 and S3) inclined relative to the surface of the support on which the capture molecules are immobilized, one (S2) being optically transparent and used for collecting light emitted from the localized areas of capture molecules and inclined by an angle of between 90 and 60° compared tot the solid support surface and the other one (S3) opposite being black or covered with a colour being black or covered with a colour having an absorption corresponding to the wavelength of the emitted light.

The present invention also protects an apparatus for performing real-time amplification and detection of target polynucleotide molecule(s) present in a sample, comprising:
a) a flow chip device comprising:
   - a flow channel having a section comprised between 0.01 and 10 mm² and a volume V1, wherein the flow channel is configured such that a solution introduced into the flow channel is cycled through the flow channel,
   - a detection chamber connected to the flow channel, said detection chamber having fixed upon one of its surface a micro-array comprising more than 4 capture molecules being immobilized in localized areas of a surface of said detection chamber, wherein said chamber has a height lower than 1 mm and a volume V2, and wherein the ratio V2/V1 is between 0.001 and 0.5,
   - at least 2 and preferably 3 different temperature regions at which temperature is regulated, each temperature region located at a different location corresponding to the flow channel, wherein one temperature region comprises the detection chamber and has a temperature allowing the hybridization of the target polynucleotide molecules to the capture molecules;
b) a holder for the flow chip device;
c) a heating system in front of the at least 2 different temperature regions;
d) a temperature controller disposed to regulate temperature within the at least 2 different temperature regions;
e) optionally a flow chip sensor;
f) an illumination light source;
g) a system operatively disposed to transport fluid through the flow channel;
h) a detector for measuring a fluorescent signal from the hybridized target polynucleotide molecules, wherein the surface of emission for a localized area is comprised between about 0.1 mm² and about 75 mm², wherein the detection of the fluorescence emitted from the localized areas of the surface having hybridized target polynucleotide molecules is assayed through an optically transparent solid support bearing the immobilized capture molecules in an observation angle which is within the forbidden angle;
wherein the different parts are integrated into the same apparatus and wherein the position of the flow chip device is fixed compared to the detector.

### Description of the drawings

The following is a description of various embodiments of the invention, given by way of example only and with reference to the figures. The figures are not drawn to scale and merely intended for illustrative purposes.

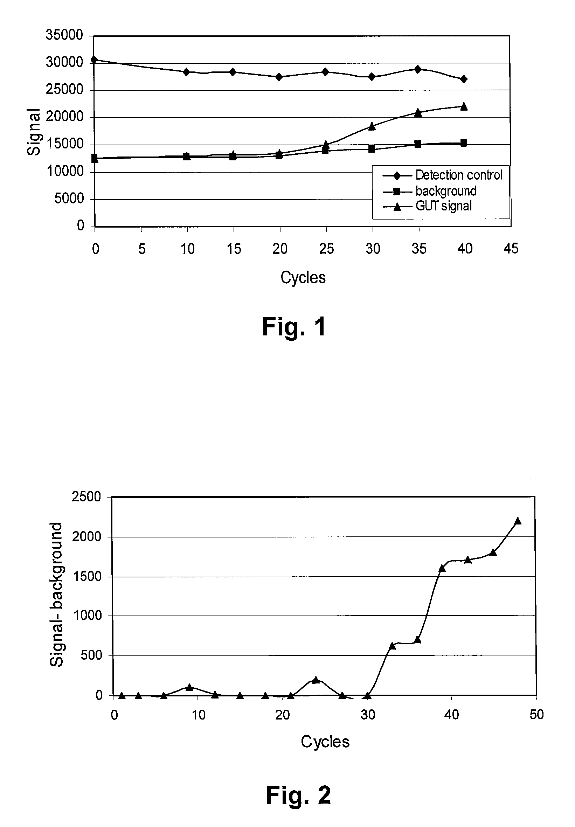

Figure 1 presents a graph showing the fluorescent signal increase of the hybridization of an amplified target (GUT) on corresponding capture probe on a microarray along the amplification PCR cycles. The PCR was performed using the flow chip device as described in the example 1. The target was present in the solution at 100 copies. Two controls were also followed during the PCR: a negative capture probe (background) and a labeled capture probe (positive detection control).
Figure 2 presents a graph showing the evolution of the fluorescent signals of amplified target DNA of S. aureus on its corresponding capture probe of a microarray along the PCR cycles after correction for the local background. The multiplex PCR was performed using the flow chip device as described in the example 2.

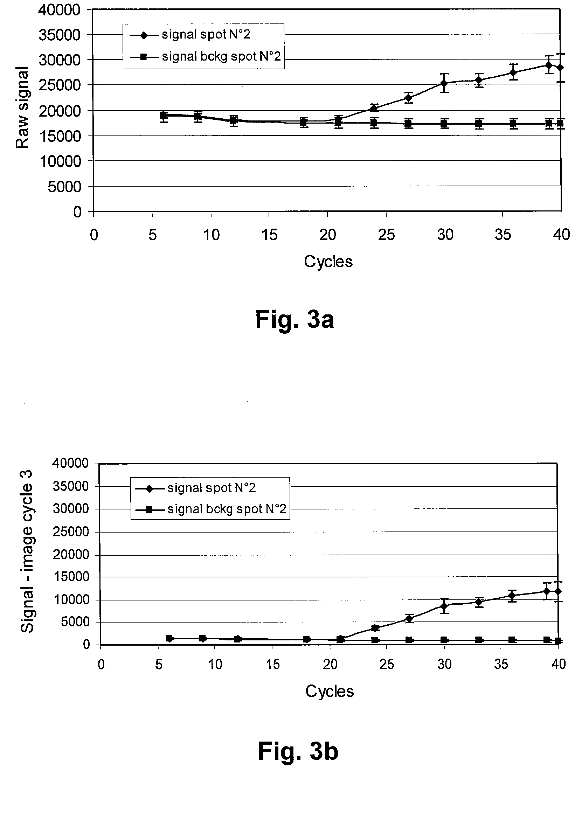

Figures 3a, 3b present graphs showing the evolution of the fluorescent signals of amplified target DNA of P35 on its corresponding capture probe of a microarray along the PCR cycles. The PCR was performed using the flow chip device as described in the example 3. Figure 3a shows the signals of the bound target P35 obtained at the different PCR cycles. In particular, the graph shows the raw values and standard deviation of the pixels of a given spot and the raw values and standard deviation for its local background. Figure 3b shows the values of signal and local background of the spot as well as the standard deviation on signal of spot and on local background when the different images at each cycle were corrected by subtraction of the image of the cycle number 3 and addition of an offset of 1500 (using "pixel math" function in Maxim DL5 software).

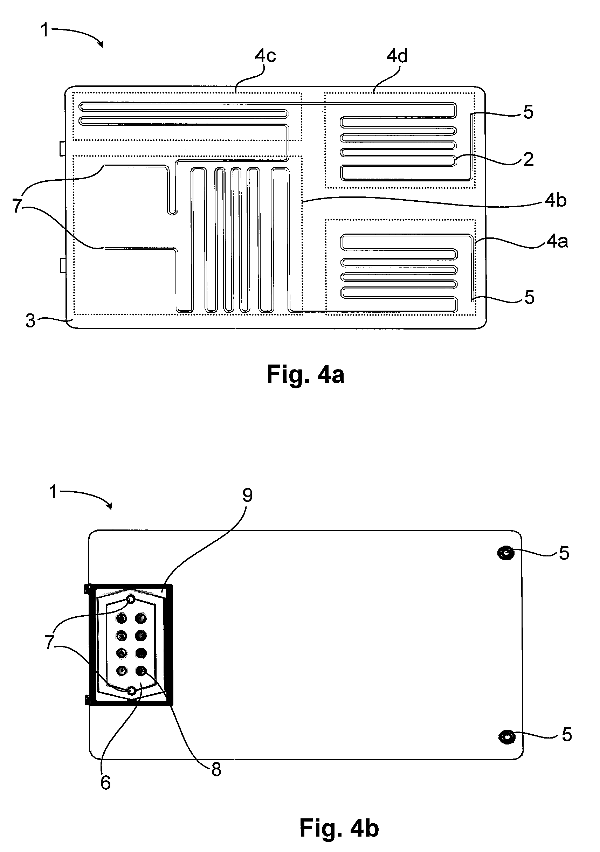

Figure 4a presents a schematic representation of a preferred integrated flow chip device 1 according to the invention. The upper side of the device comprises a flow channel 2 which is disposed within a substrate 3. The flow channel 2 comprises four different temperature regions 4 (4a for denaturation, 4b and 4c for annealing/hybridization and 4d for elongation) which are separated from each other in order to permit heating at different temperatures by an external heating system (not shown). The device also comprises ports for the pump 5 and connective ports 7 between the channel and the detection chamber.
Figure 4b schematically shows a view of the bottom side of the device 1. The device 1 comprises two extremities of the flow channel comprising ports 5 for connecting the channel to a peristaltic pump (not shown) and for introducing a sample solution. The flow channel is connected to a detection chamber 6 by connective ports 7. Capture molecules of a micro-array 8 are immobilized on the surface of an optically transparent solid support 9. The design of the integrated flow chip device is described in the example 4.

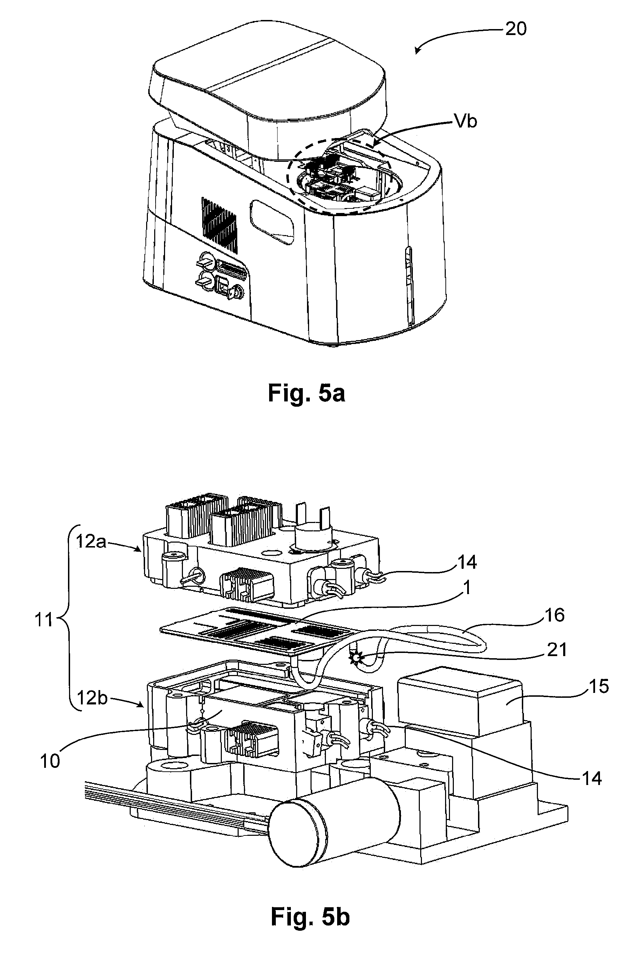

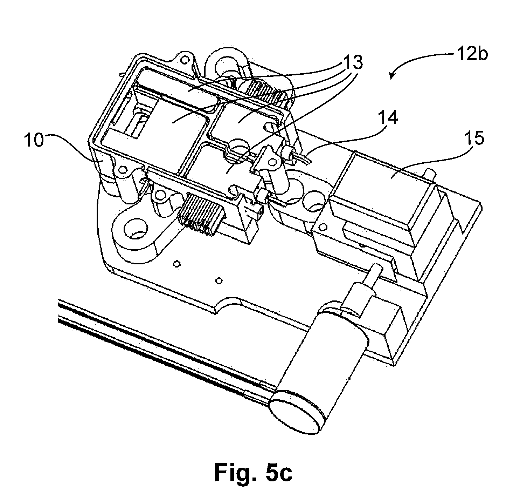

Figure 5 shows a schematic representation of a preferred apparatus 20 according to the invention. An enlargement of the instrument showing the heating system 11, its regulation and the system disposed to transport fluid through the flow channel (pump) is presented in Figures 5b and 5c. The apparatus 20 comprises a holder 10 for the flow chip device 1, and a heating system 11 comprising an upper part 12a and a lower part 12b. The upper and lower parts 12a, 12b are composed of a number of heating blocks 13, in this exemplary embodiment equal to four, which are positioned in front of the different temperature regions of the flow chip device 1 and screwed together. The temperature of the heating system is regulated by a temperature controller 14. There is one temperature controller 14 for individual regulation of the temperature of each heating block 13. The flow chip device is connected to the peristaltic pump 15 through pump tubing 16. A flow chip sensor 21 is fixed on the pump tubing for measuring the passage from gas to liquid phase. The flow chip instrument (F-RAP) is also described in the example 5.

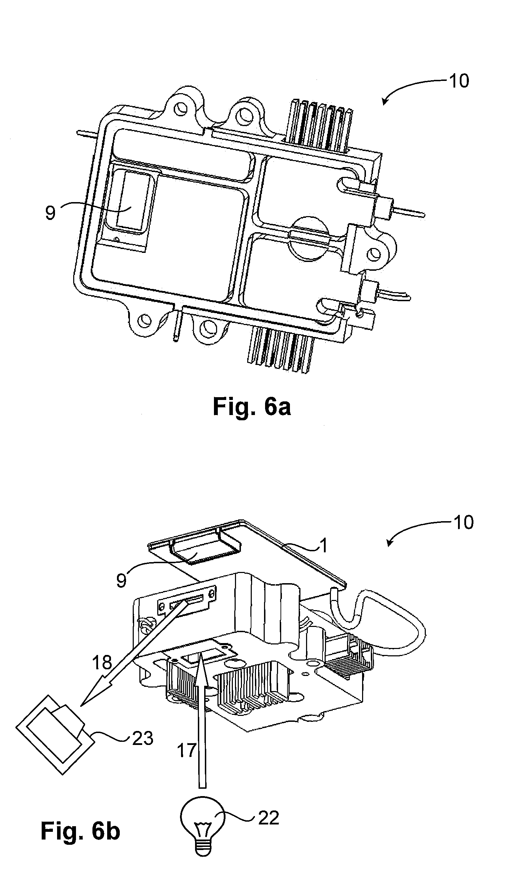

Figures 6a, 6b show a schematic top view and bottom view of the holder 10 of the flow chip device 1 respectively. Once positioned in the instrument, the detection chamber has its external optical block 9 surface facing a direction from which it receives a light illumination beam 17 from a light source 22 and a side surface facing the detector 23 through a slit so that excitation light of the micro-array surface is detected within the forbidden angle of the light emission 18.

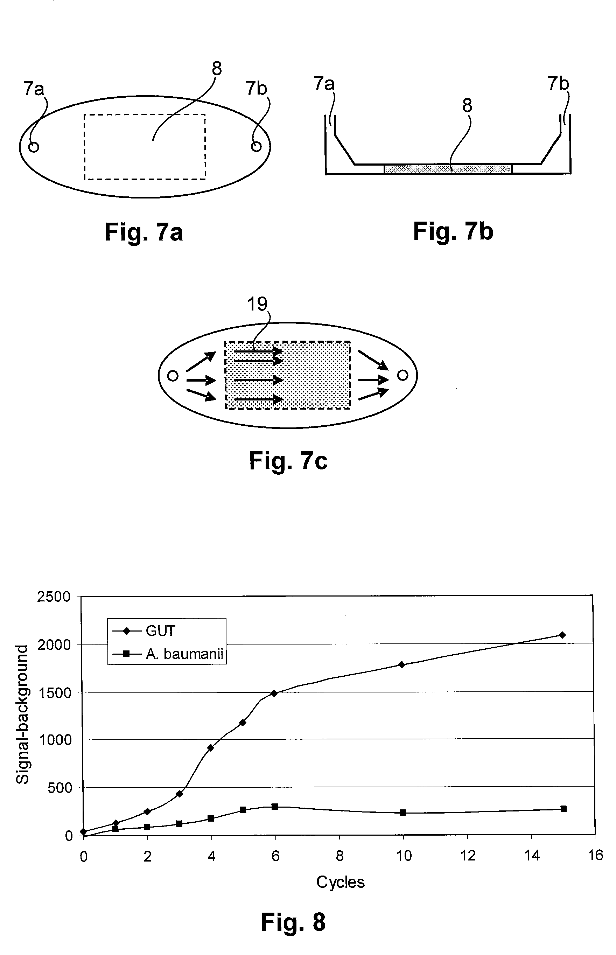

Figures 7a-7c show a schematic presentation of a preferred detection chamber according to the invention. Figure 7a schematically shows a front view of the geometry of the chamber. Figure 7b schematically presents a transversal view of the chamber having different heights in dependence of the location along the longitudinal axis of the chamber. Figure 7c schematically presents the liquid flux in the chamber between the connective ports 7, where port 7a represents the injection connective port, port 7b represents the output connective port, and the micro-array is denoted with reference number 8. The flux direction of the liquid on the micro-array side is presented by reference number 19.
Figure 8 presents a graph showing the evolution of the fluorescent signals which accumulates along the cycles when a solution of a fixed amplicons concentration is cycled in an integrated fluidic system. The fluorescent signal is corrected for the local background. An embodiment of the flow chip integrated device is described with reference to figure 4 and an embodiment of the flow chip instrument (F-RAP) with reference to figures 5 and 6. The amplicons GUT and A. Baumanii were introduced at two different concentrations into the flow device in the hybridization buffer and the solution was cycled through the flow channel and the detection chamber between the different temperature regions necessary for PCR. The hybridizations were checked on the micro-array after each cycle. Accumulation of the signal of the two amplicons at different concentrations was observed. The experiment is described in example 6.

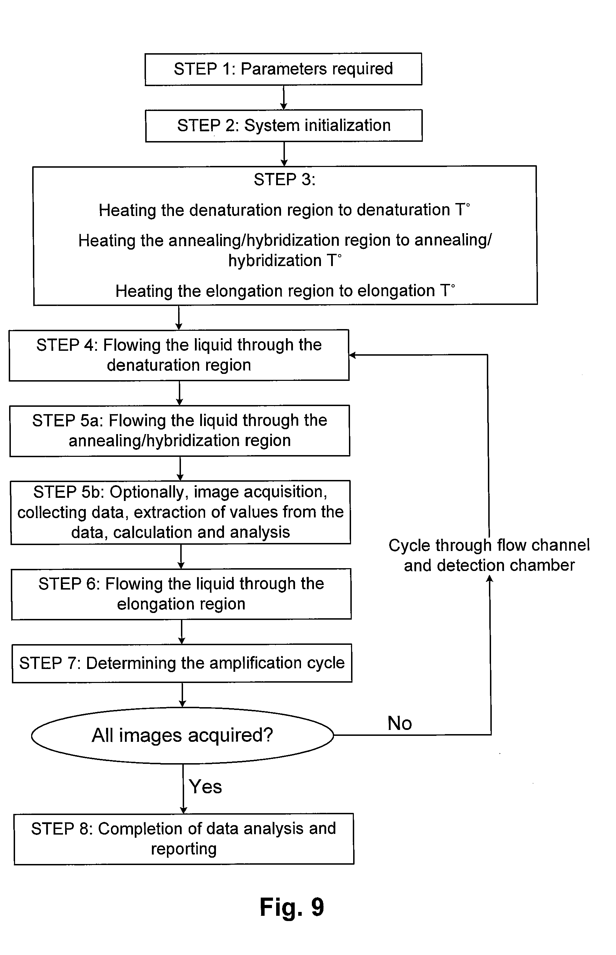

Figure 9 represents a general flow chart of the different steps of the process according to the invention for making the real-time detection together with different cycles of a PCR in a microfluidic system. This general flow chart corresponds to the embodiment in which real-time PCR apparatus is controlled by a programmable computer as provided in the example 7 and is explained in the text of the invention.

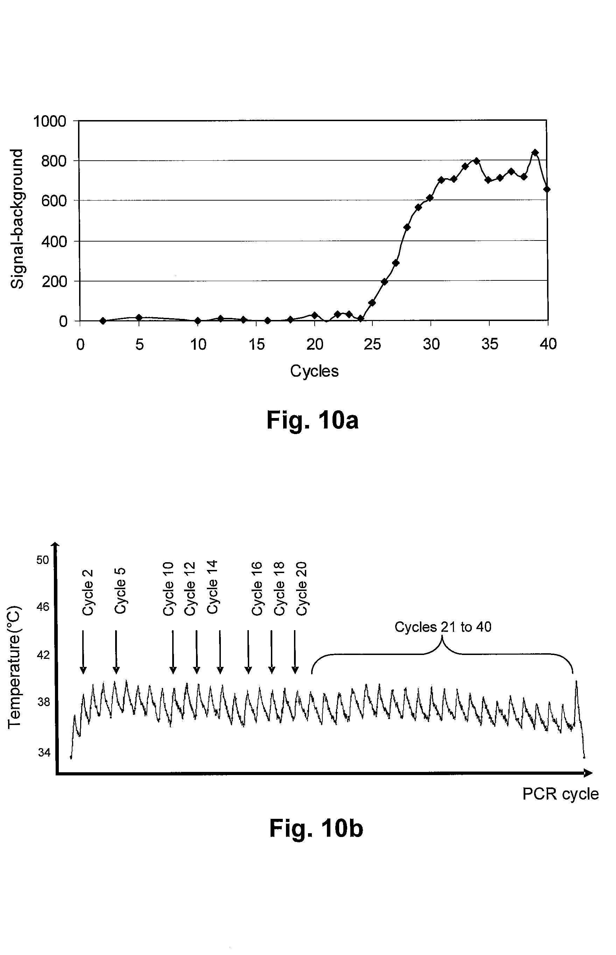

Figure 10a presents a graph showing the evolution of the fluorescent signals of amplified target DNA of H. influenzae on its corresponding capture probe of a microarray along the PCR cycles after correction for the local background. The multiplex PCR was performed using an integrated flow chip device as discussed with reference to figure 4 and the flow chip instrument (F-RAP) discussed with reference to figures 5 and 6. The experiment is described in example 7. Figure 10b shows a display of the amplification cycle counting using the thermocouple probe. The arrows indicate at which cycles a measurement of the fluorescent signals is performed. From cycle 21, the signal detection was performed at each cycle.

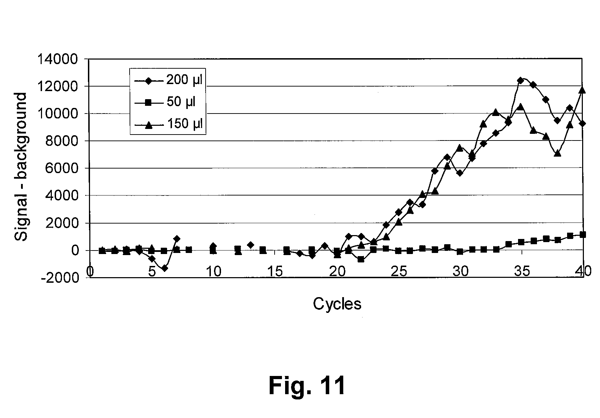

Figure 11 presents a graph showing the effect of the volume of PCR solution on the evolution of the fluorescent signals of amplified target DNA of H. influenzae on its corresponding capture probe of a microarray along the PCR cycles after correction for the local background. The multiplex PCR was performed using an integrated flow chip device as discussed with reference to figure 4 and the flow chip instrument (F-RAP) as discussed with reference to figures 5 and 6. The total volume of the flow device including the detection chamber was 240 µL. The experiment is described in example 8.

### Definitions

Unless otherwise defined, all technical and scientific terms used herein have the same meaning as commonly understood by one person ordinary skilled in the art to which this invention belongs.

The terms "nucleotide sequence, micro-array, target (and capture) nucleotide sequence, bind substantially, hybridizing specifically to, background, quantifying" are as described in WO 97/27317, which is incorporated herein by way of reference.

The terms "nucleotide triphosphate, nucleotide, primer sequence" are those described in the European patent application EP1096024 incorporated herein by reference.

The term "gene" means fundamental physical and functional unit of heredity, which carries information from one generation to the next; a segment of DNA located in a specific site on a chromosome that encode a specific functional product. The DNA segment is composed of transcribed region and a regulatory sequence that makes transcription possible (regions preceding and following the coding DNA as well as introns between the exons).

The term "locus" means the position of the single nucleotide polymorphism (SNP) upon the sequence of the gene.

"Homologous sequences" mean nucleotide sequences having a percentage of nucleotides identical at corresponding positions which is higher than in purely random alignments. Two sequences are considered as homologous when they show between them a minimum of homology (or sequence identity) defined as the percentage of identical nucleotides found at each position compared to the total nucleotides, after the sequences have been optimally aligned taking into account additions or deletions (like gaps) in one of the two sequences to be compared. The degree of homology (or sequence identity) can vary a lot as homologous sequences may be homologous only in one part, a few parts or portions or all along their sequences. Nucleotide sequences differing by only one base are sequences highly homologous and qualified as single nucleotide polymorphisms (SNPs). The parts or portions of the sequences that are identical in both sequences are said conserved. Protein domains which present a conserved three dimensional structure are usually coded by homologous sequences and even often by a unique exon. The sequences showing a high degree of invariance in their sequences are said to be highly conserved and they present a high degree of homology.

Methods of alignment of sequences are based on local homology algorithms which have been computerised and are available as for example (but not limited to) Clustal®, (Intelligenetics, Mountain Views, California), or GAP®, BESTFIT®, FASTA® and TFASTA® (Wisconsin Genetics Software Package, Genetics Computer Group Madison, Wisconsin, USA) or Boxshade®.

The term "consensus sequence" is a sequence determined after alignment of the several homologous sequences to be considered (calculated as the base which is the most commonly found at each position in the compared, aligned, homologous sequences).

The consensus sequence represents a sort of «average» sequence which is as close as possible from all the compared sequences. For high homologous sequences or if the consensus sequence is long enough and the reaction conditions are not too stringent, it can bind to all the homologous sequences. This is especially useful for an amplification of homologous sequences with the same primers called, consensus primers. Experimentally, the consensus sequence calculated from the programs above can be adapted in order to obtain such property.

"Micro-arrays and arrays" mean solid supports on which single capture probes or capture probes species are immobilized in order to be able to bind given specific targets preferably protein or nucleic acid. Micro-arrays are preferentially obtained but not limited to deposition of the capture molecules on the substrate done by physical means such as pin or pin and ring touching the surface, or by release of a micro-droplet of solution by methods such as piezo or nanodispenser. Alternatively, *in situ* synthesis of capture molecules on the substrate is one of the invention's embodiments with light spatial resolution of the synthesis of oligonucleotides or polynucleotides in predefined locations such as provided by US-P-5,744,305 and US-P-6,346,413. The micro-array is preferentially composed of spots of capture probes deposited at a given location on the surface or within the solid support or on the substrate covering the solid support. However, capture probes can be present on the solid support in various forms being but not limited to spots. The spatial distribution of the spots on the solid surface on which the capture probes are attached is preferably but not limited to a geometrical order for example in square, rectangular or even as a single row of spots. One particular form of application of micro-array is the presence of capture probes in wells having either one or several different capture probes per well and being part of the same support. Advantageously, micro-arrays of capture probes are also provided on different supports as long as the different supports contain specific capture probes and are distinguished from each other in order to be able to allow a quantification of a specific target sequence. This is achieved by using a mixture of beads having particular features and being able to be recognized from each other in order to quantify the bound molecules.

The terms "capture molecule" relate to molecules capable to specifically bind to a given polynucleotide or polypeptide or protein or to a family thereof. Preferably, polynucleotide binding is obtained through base pairing between two polynucleotides one being the immobilized capture probe or capture sequence and the other one being the target molecule (sequence) to be detected.

The term "incident angle" represents the angle between a direction incident on a surface and the line perpendicular to the surface at the point of incidence, called the normal. In the present invention, the incident angle is considered in(side) the support since the emitted light is detected going through the support.

The term "critical angle" in the present invention is the angle given in degrees in the support relative to the normal versus the solid support surface defined by θ_{c}= sin⁻¹(n₂/n₁), where n₁ is the refractive index of the solid support and n₂ is the refractive index of the outside. In the present invention, n₂ is preferably a water solution (n₂∼1.33). The critical angle is the value of the incidence angle at which total internal reflection in the support occurs. The critical angle can be calculated and expressed in radians in the same way.

The term "observation angle" (θobin) is the angle used for the observation of the emitted light in the support and is expressed relative to the normal in the support versus the solid support surface bearing the target molecules.

The term "θobout" is the observation angle for the detection device located outside the support relative to the normal of the side surface of the transparent optical block.

The "forbidden angle" of the invention is an angle comprised between the critical angle and 90° for a light beam of a wavelength corresponding to an emitted light present from a given solution and detected through a given support.

The term "evanescent wave coupling" or "evanescent coupling" is a process by which electromagnetic waves are transmitted from one medium to another medium by means of the evanescent (or decaying) electromagnetic field(s).

In its common meaning, the term "evanescent field" or "evanescent wave", refers to an exponentially decaying electromagnetic field generated on the far or distal side of a totally internally reflecting interface that is illuminated by an incident light source. The evanescent wave gives an excitation energy which is the same as the energy of the wavelength of the incident light that was totally internally reflected. This energy allows the excitation of molecules fixed on the surface where the total internal reflection occurs (Induced evanescence). The evanescent field propagates with significant energy for only a relatively short distance from the distal surface of the interface (e.g., in the order of magnitude of its wavelength).

The term "emitted evanescence" or "reverse evanescence" is the reverse of induced evanescence, i.e. the process by which light emitted from objects very close (within one or few wavelengths) to the far side of a totally reflecting surface (outside the support) is transmitted to the near side (inside the support).

The term "optically transparent support" means a support which has the features for conducting the light with a very low absorption and without bringing defects into the homogeneity of the light beam. Preferably, "Optically transparent support" means a support allowing at least 90%, preferably at least 95% and even more preferably at least 99% of the light to go through. Typical optical transparent support is made of high grade quality glass or material such as Zeonex or Topas.

Fluorescent label includes fluorescent labeled nucleotides which are incorporated into the amplification product. This is either achieved by using fluorescent labeled nucleic acid primers or labeled deoxyribonucleotides. Fluorescent label also includes intercalating fluorescent dyes like SYBR Green.

The term "real-time PCR" means a method which allows detecting and/or quantifying the presence of the amplicons during the PCR cycles. In the real-time PCR, the presence of the amplicons is detected and/or quantified in at least one of the cycles of amplification. The increase of amplicons or signal related to the amount of amplicons formed during the PCR cycles is used for the detection and/or quantification of a given nucleotide sequence in the PCR solution.

The term "amplicon" in the invention relates to the copy of the target polynucleotide molecules being the product of enzymatic nucleic acid amplification.

"Biological target molecule" means a molecule which is involved in biological processes. Target molecules are limited to nucleic acids.

### Detailed description of the invention

The present invention proposes a full solution for fast real-time amplification and online detection and/or quantification of multiple polynucleotides in a fully automated close device. The invention solved the problems by giving specifications to the different parts of the system working together and by specific features of the detection chamber having the immobilized capture molecules. The invention is best performed in an instrument adapted for a device which includes the different features and controls and with the particular parameters for performing the assay in such a device. Such an instrument is controlled by appropriate software which incorporates the measurement needed for the assay and the analysis of the data and/or the quantification of the target(s) present in the solution.

The method and device are for the detection of possible multiple targets. Possible means that the assay is able to detect them when they are present in a sample. However, in the most common situation only one or a limited number of these targets are present in a given sample. The method and device detect one and better at least 4 and even better more than 10 different targets when present in a given sample.

The overall assay is performed in a very fast way with one cycle of amplification being performed in less than 3 min and preferably in less than 2 min and even in less than 1 min so that a typical amplification/detection experiment of 35 cycles is performed within 105 min and even 70 min and even better within 35 min. The speed of the assay was unexpected in such a complex assay since the time of the hybridization for detection of the amplified target is very short preferably being less than 1 min or even less than 30 sec or better less than 20 sec for a cycle. Generally, the time will be about 2 sec or more, preferably 5 sec or more. As exemplified in example 4 for a flow device having a capacity of about 240 µL, the time of the passage of the 50 µL solution in the micro-array detection chamber takes about 16 sec per cycle. The typical hybridization time for a micro-array is usually longer than a few hours in a static micro-array like 14-16 h in a standard protocol (Ideker et al 2002, Hybridization and post-hybridization washing, in "DNA Microarrays" D. Bowtell and J. Sambrook, cold spring Harbor Laboratory Press, Cold spring Harbor, New York. Pg 228-239). The reaction is enhanced by dynamic hybridization based on chaotic mixer which improves both the speed of micro-array hybridization and the signal intensities as compared to static hybridization (McQuain et al, 2004, Analytical Biochemistry, 325, 215-226). However even with dynamic hybridization the time needed is one hour and even longer and of the order of 5-20 min in a flow through assay (Mocanu et al 2008, Anal. Biochem., 380, 84-90). This feature makes the present assay competitive with the fast PCR performed with detection in solution with the advantage being a multiple target real-time amplification/detection due to the use of micro-array with multiple capture probes. This results from the overall features that have been proposed as part of a complete solution in the present invention.

One feature for short reaction time is the fact that the overall solution passes onto the same micro-array at each cycle since the micro-array is part of the flow system. Also preferably the volume of the detection chamber is limited and is preferably configured to contain a volume of solution comprised between 1 µL and 1 mL and preferably between 1 and 20 µL and even better between 1 and 10 µL. Preferably, the height of the reaction chamber is lower than 1 mm and preferably lower than 250 µm. The height of the liquid in the detection part of the detection chamber is preferably comprised between 10 and 250 µm and preferably between 50 and 150 µm Also the different solid parts of the flow device are kept at given temperatures. Only the solution having a limited volume is heated and cooled along the cycles resulting in a fast amplification cycle.

Having a short hybridization time has an unexpected advantage which is to work with a small solution volume. The small volume is easily heated or cooled when moving from one region to the other according to the requested temperatures needed for the different amplification steps since it has a low heat capacity. Also the small volume allows the target taken from a sample to be in a concentrated solution thus increasing the sensitivity of the method. Dilution of the sample in a large volume will increase the time of hybridization but this is not require as shown here above.

Another advantage of the present invention is the fact that the detection is fully integrated into a continuous process. There is no special separate step of hybridization which requires handling of the device and time as for the step by step PCR /micro-array described in prior art.

The present method allows accumulation of the target to be hybridized on the micro-array at each cycle of contact between the solution and the capture molecule. Unexpectedly, the hybridized targets are stable on their capture molecules even at the rather high hybridization temperature and also when the liquid phase has been removed, causing the capture probes to be in a gas phase. The capture molecules are also still able to fix in a very efficient way the targets in the next cycle after staying at a rather high temperature in the absence of solution. One feature of the present invention is the accumulation of the hybridized amplicons onto specific spots along the amplification cycles as multiple amplification cycles share a common micro-array surface. The temperature and the solution composition are chosen in such a way as to favour the specific hybridization of a given target amplicon onto its specific capture molecule present in a given discrete region (spot) of the micro-array. Effects of the solution composition to favour or disfavour the hybridization of a given polynucleotide sequence on another polynucleotide sequence include the stringency of the solution or the presence of specific compound such as urea, or detergents.

The conditions are best chosen as to allow hybridization but not removal of the hybridized amplicons between successive cycles in order to accumulate the hybridized amplicons on a specific spot and thus increase the sensitivity of the detection of the method. This feature makes the present method to be particularly sensitive even with a very short contact between the solution and the capture molecules.

Another problem linked to the PCR which is solved is the formation of bubbles during the heating at high temperature in the denaturation step and the change of pressure due to the variation in the temperatures of the different phases of the fluidic process. In the present invention, the overall system is preferably present in two phases, preferably a liquid phase and a gas phase. Bubbles when formed mostly join the gas phase and do not interfere with the liquid phase so as to allow homogenous hybridization of all spots. Also the increase pressure due to liquid expansion at high temperatures is amortized by the presence of the gas phase.

In this preferred embodiment, the present invention also solves the problem of heterogeneity of the fluidic having two phases which position changed with time by providing a chip sensor for detection of the solution position or presence or its flow or the liquid/gas transition phase and thus allowing the control of the timing for detection of the array either in the presence of solution or in the gas phase. The sensor signal is analyzed in order to detect changes associated with the presence or movement or flow of liquid and preferably the detection of the transition phase within the flow and/or the passage of the front or end of the solution. Timing for the presence of the liquid in the detection chamber is preferably deduced from the analysis of different parameters including the position of the sensor on the flow chip, the timing of the signal change and the flow rate of the liquid. The volume of the liquid is used in order to determine the duration of the liquid present into the detection chamber. Example of velocity determination of the flow is presented in GB2433259 using the absorbance of the DNA under illumination of the flow channel with UV light.

Also the sensor allows the counting of the cycle number by determination of the number of variations in the sensor recording with time linked to the passage of liquid at the sensor location. Preferably, the number of transition phase of liquid to gas corresponds to the number of cycles. If necessary, corrections are introduced into the calculation by first standardized the data processing. Counting the cycles allows providing a relationship between the spot values and the amplification cycles which is one requirement for real time amplification and/or quantification. Preferably the quantification of the target(s) is performed by expressing the spot value corresponding to the target detection as a function of the cycles of amplification.

In a preferred embodiment, the measurement of a fluorescent signal from the hybridized target polynucleotide molecules is performed in a gas phase as that may lower the background signal from the solution.. In an alternative embodiment, the measurement of a fluorescent signal from the hybridized target polynucleotide molecules is performed in the presence of the amplification solution containing the labeled target polynucleotide molecules. Assay of hybridized target in the presence of solution having a homogeneous phase gives comparative signals for the localized area. Preferably, the detection is performed in the presence of a homogeneous solution phase.

It was another unexpected result that it is possible to obtain a detection of the amplified targets during the cycles and in the presence of the solution containing high amount of fluorochrome label which is incorporated into or bound to the target during the amplification.

One of unexpected result of the present invention is the very high stability of the probes. This stability is exemplified in figure 1 where the signals of the detection control spots are very stable along the cycles even if they were subjected to various successive illuminations which lead to some bleaching. This method clearly differentiated from the prior art (RAP technology) on this feature. The stability of the detection controls is a clear indication of the stability of the capture molecule along the process and justifies the quantitative comparative analysis of the signals obtained on the different capture molecules.

One of the problems specific of the micro-array is the fact that all the spots or localized area having immobilized capture molecules have to be treated the same way in all steps of the process in order for the results to be relevant for the different capture molecules present in different locations of the micro-array. The present invention solves the problem linked with the continual passage of the liquid on the micro-array. Preferably at each of the amplification cycles, the liquid is in contact with the different localized area for the same amount of time so as to allow the same hybridization time for all spots and for each cycle. Preferably the standard deviation of the signals obtained from replicate spots located at different positions on the micro-array surface is lower than 20% and even lower than 10% of the test value.

Positioning of the optical block relative to the detector is preferably made once (which may be checked once in a while, like every month; or - if more variation is expected - once for every flow-chip, or any time in between) and in order to obtain the best surface resolution of the micro-array. The thickness of the optical block having the capture molecule is preferably of at least 0.5 mm, preferably 1 mm and more preferable 3 mm or more, and even about 5 mm or more. The present invention has an unpredicted advantage of allowing the optical block to be a thick one and so allowing the detection in the forbidden angle to be easier than with a thin block. This is possible given the fixed temperature of the detection chamber. Generally, the thickness will be less than 20 mm, preferably less than 10 mm.

Another feature which was not envisaged is the position stability of the detection system compared to the surface to be detected covered with localized area having different capture molecules. This physical stability leads to images of the micro-array surface along the cycles which are perfectly superposed to each other having the same geometry so that the spots are at the same position and the different images are easy to compare and to analyze. In one of the preferred embodiment, the gridding of the micro-array image is performed once on the first image taken and the same grid is then used for the following images so as to be perfectly compared. This permits to avoid different gridding for different images of a same experiment and difficulties in data comparison. Also images are easily superposed or subtracted without complex correction of the pixels localization.

A side feature of the present invention is that the liquid is cycled through the flow channel and micro-array which is a close system. Close system has the advantage of preventing the contamination of the amplified product in the surrounding environment and avoid contamination for further assays. Once the assay is done the full device is removed from the instrument and discarded as such without opening. However, if needed, further reading of the presence of the hybridized targets on their capture molecules is performed like for control or verification of the assay.

### Chamber and flux

In a particular embodiment of the device and method, the detection chamber has a shape selected from: elliptic, oval, rhomboidal, hexagonal, octagonal, diamond. The chamber is configured in order to produce a homogenous repartition of the liquid flow onto the micro-array. Particularly the geometry of the chamber takes into account the flux of the liquid, the height of the chamber and its ratio width/length for the design of the chamber in order for the liquid influx to be homogeneously distributed onto the micro-array surface and the liquid outflux to be also homogeneously removed from the chamber so that no liquid is left into the chamber during the gas phase of the flow through the detection chamber. The chamber is configured taking into consideration the angle of the liquid in the microchannel compared to the chamber main orientation, the flow rate of the liquid, the height of the chamber and the hybrophilicity/hybrophobicity of the chamber surface. When the chamber surface is highly hydrophilic, the solution flows into the chamber smoothly and rapidly without generating air bubbles. The hybdrophilicity/hybrophobicity is best measured by the contact angle of the solution-gas interface on the solid surface. The surface of the detection chamber carrying the micro-array has a contact angle preferably lower than 100°, better lower than 80° and even better lower than 60°.

A preferred geometry of the chamber is presented in figure 7a. Preferably for a given chamber, and micro-array surface having a particular hydrophobic property and a given flow rate, the width of the chamber and the height of the liquid are adjusted to obtain a liquid/gas front homogeneously distributed along the width of the chamber as illustrated in figure 7b. In a preferred embodiment, the orientation of the influx inside the chamber is best at 0° compared to the chamber longitudinal axe and the chamber sides are symmetrical compared to the liquid flux. In still a preferred embodiment, the injection part of the chamber has a height adjusted in order to have a section equal to the section inside the chamber as outlined in figure 7c.

The detection chamber has in some embodiments a channel-like form having either a coil shape or being straight. In this particular embodiment, the micro-array is a linear array which is spotted inside the channel.

Preferably all the localized areas are covered by the solution when going into the chamber for the same amount of time. Also preferably the flow of the liquid on each localized area is identical or does not vary by more than 20 % and even better by 10 % from one localized area to the other. Reproducibility of duplicate spot values is best way to control the homogeneous repartition of the liquid in the flow chamber. Duplicate spots values preferably do not vary more than 20% and better no more than 10% along the cycles of amplification. In a particular embodiment, the flow in the detection chamber is a laminar flow and the Reynolds number of the flow is lower than a fixed value which differentiates the laminar from the turbulent flow. More precisely, the Reynolds number is lower than about 2300.

In still another embodiment, the flow chip device is configured to contain a volume of solution comprised between 20 µL and 2 mL.

The cycling of the solution is obtained by repeatedly transporting the solution through the flow channel.

The amplification solution is transported unidirectional or bidirectional between the temperature regions of the flow chip device.

In some embodiments, the amplification solution is transferred between the temperature regions in a circular manner (unidirectional) (figure 4) or in a back-and-forth manner (bidirectional). Various means for transporting the solution through the channel exist. The transport is either an active transport, e.g. by applying a pressure or a passive transport, e.g. diffusion or transportation driven by capillary forces. Thus, when the amplification solution is passed through the temperature regions, this implies an active as well as a passive transport through the regions. The transportation system in this context is e.g. a passive system inherently comprised in the flow chip device, e.g. in cases where the transport is solely based on physical effects such as convection or diffusion.

Active transportation in microfluidic device relies on one of the two manners of fluid transport: pressure-driven or electrokinetically-driven Flow.

Electrokinetic transport refers to the combination of *electroosmotic* and *electrophoretic* transport. Electroosmosis refers to the *bulk* movement of an aqueous solution past a stationary solid surface, due to an externally applied electric field. Electroosmosis requires the existence of *a charged double-layer* at the solid-liquid interface.

Electrophoresis describes the motion of a charged surface submerged in a fluid under the action of an applied electric field.

Preferably, cycling of the solution in the flow chip device is obtained by repeatedly transporting the solution through the flow channel using pressure-driven flow comprising but not limited to: the action of a pump, a compressing element, the use magnetic beads and the like or combinations thereof.

The pump is preferably a peristaltic pump having a rotation speed comprised between 1 and 500 rpm. The rate of liquid flow in the flow channel is preferably comprised between 6 µL/min and 3000 µL/min and preferably between 50 and 500 µL/min. In still a particular embodiment, the rate of liquid flow is changed according to the timing of the experiment and the position of the liquid in the flow device. Adjustment of the liquid flow is made to obtain a given time of the contact between the liquid and the region in order to adjust the timing a given temperature being optimal for the amplification process being for PCR the denaturation, annealing, and elongation temperature steps.

In a particular embodiment, the liquid flow is stopped in a particular region of the flow device for a given time and at a particular cycle of the amplification. In PCR, the liquid is stopped for a given time at the first cycles in the denaturation region in order to obtain proper denaturation of genomic DNA necessary for starting the first annealing step. In still a particular embodiment, the detection chamber is at the same temperature as the annealing region and is then considered as part of the annealing step of the PCR cycle.

The surfaces of the flow chip in contact with the amplification solution are preferably coated with a blocking reagent to reduce the affinity of the surfaces for the target DNA and the PCR reagents (primers, dNTP, DNA polymerase).

The blocking reagent is preferably selected from the group consisting of: Tween-type surfactants (Polysorbates, Sorbitan esters, poly(oxy-1,2 ethanediyl) derives, Tweens), Triton X-100 (polyethylene glycol p-(1,1,3,3-tetramethylbutyl)-phenyl ether, (octyl phenol ethoxylate, polyoxyethylene octyl phenyl ether, 4-octylphenol polyethoxylate, Mono 30, TX-100, t-octylphenoxypolyethoxyethanol, Octoxynol-9), Polyethylene glycol (PEG) and serum albumin (human serum albumin, bovine serum albumin or BSA).

Tween-type surfactants (Polysorbates, Sorbitan esters, poly(oxy-1,2 ethanediyl) derives, Tweens) are water soluble non-ionic polymeric detergent comprised of complex esters and ester-ethers derived from hexahydric alcohols, alkylene oxides and fatty acids by adding polyoxyethylene chains to hydroxyl of sorbitol and hexitrol anhydrides (hexitans and hexides) derived from sorbitol and then partially esterifying with the common fatty acids such as lauric, palmitic, stearic and oleic acids.

In one embodiment the Tween-type surfactant is selected from one or more of Tween 20, Tween 40, Tween 60 or Tween 80, also known in the pharmaceutical industry as polysorbate 20, polysorbate 40, polysorbate 60 and polysorbate 80. Polysorbate 20 (Polyoxyethylated Sorbitan Monolaurate) is a laurate ester, Polysorbate 60 (Polyoxyethylated Sorbitan Monostearate) is a mixture of stearate and palmitate esters; and Polysorbate 80 (Polyoxyethylated Sorbitan Monooleate) is an oleate ester. Such Tween type surfactants are commercially available and/or are prepared by techniques known in the art.

In a preferred embodiment the Tween-type surfactant is Polyoxyethylated Sorbitan Monolaurate (polysorbate 20, Tween 20).

The concentration of the blocking reagent is preferably comprised between 0.01 and 2%. Coating is preferably done but not restricted to protein such as BSA (serum albumin bovine) used as coating agent in concentrations ranging from 0.01% to 0.05%. High molecular weight molecules like PEG (Polyethylene glycol) is also used as coating agent in concentrations ranging from 0.05% to 2%. Detergents like Tween 20 or Triton X100 have a coating effect when used at concentrations of about 0.1 %.

In a preferred embodiment, the blocking reagent is part of the reagents for polynucleotide molecule amplification, said blocking reagent being preferably selected from the group consisting of: Tween-type surfactant, Triton X-100, PEG and serum albumin.

Alternatively, a pre-coating with the blocking reagent is performed before the amplification. The blocking solution is circulated through flow channel and reaction chamber of the flow chip device. In an embodiment, the surface of the flow chip in contact with the solution is pre-coated between 1 and 90 min before the amplification with a blocking reagent selected from the group consisting of: Tween-type surfactant, Triton X-100, PEG and serum albumin. In a yet alternative embodiment, the flow channel and flow chip may be pre-treated by the manufacturer of the flow chip, and the flow chip can be delivered ready to be used.

The present invention in a particular embodiment covers a method of assay of a phase transition within a microfluidic device for follow up real-time PCR cycles comprising the steps of:
a) providing a flow chip device comprising:
   - a flow channel having a section comprised between 0.01 and 10 mm² and a volume V1, wherein the flow channel is configured such that a solution introduced into the flow channel is cycled through the flow channel,
   - a detection chamber in fluid communication with the flow channel, said detection chamber having an optically transparent solid support comprising at least one capture molecules being immobilized in localized areas of the surface of said transparent solid support, wherein said chamber has a height lower than 1 mm and a volume V2,
   - at least 2 and preferably 3 different temperature regions at which temperature is regulated, each temperature region being located at a different location of the flow channel, wherein one temperature region comprises the detection chamber and has a temperature allowing the hybridization of the target polynucleotide molecules to the capture molecules,
b) a flow chip sensor for the detection of a phase transition of liquid/air and/or air/liquid,
c) introducing a solution having a volume V3 and containing target polynucleotide molecules into the flow channel and reagents for polynucleotide molecule amplification, wherein the ratio V3/(V1+V2) is higher than 0.02 and lower than 1 (so that air is present in the device beside the solution),
d) submitting the solution to at least 5 amplification cycles to obtain labeled target polynucleotide molecules, wherein one amplification cycle is obtained by cycling the solution through the flo, channel and the same detection chamber between the different temperature regions and wherein a amplification cycle is performed in less than 3 min,
e) determining the amplification cycles by data analysis of the flow chip sensor,
f) measuring a fluorescent signal at different cycles of the amplification from the hybridized target polynucleotide molecules in at least 3 and preferably in at least 5 different amplification cycles by detecting the fluorescence emitted from the localized areas of the surface having hybridized target polynucleotide measured after excitation of the fluorochrome by a light beam,
g) analyzing the signal values obtained from the localized area along the amplification cycles in order to detect and/or to quantify the target polynucleotide molecule(s) present in the sample.

Particular application also comprises a micro-array in the detection chamber, said micro-array comprising more than 4 and preferably more than 20 capture molecules being immobilized in localized areas of the surface of a solid support.

The amplification solution preferably provides two phases in the flow channel being a gas phase and a liquid phase. In a particular embodiment, the two phases of the flow system are composed by the water based solution of the amplification and an oil phase. The ratio V2/V1 is preferably comprised between 0.001 and 0.5 and better between 0.01 and 0.1.

Unexpectedly, a target amplicon was detected at an earlier cycle when the flow chip device was nearly completely filled with the amplification solution (V3) as compared to the use of a small volume as provided in figure 11. Preferably, a gas phase remained in the closed device even if it is very small. The presence of a gas phase allows physical separation of the liquid from one cycle to the other and an easy counting of the amplification cycles with a sensor detecting the transition phase at each cycle.

In a preferred embodiment, the ratio V3/(V1+V2) is higher than 0.5, preferably higher than 0.75, even higher than 0.9 and lower than 1. The ratio V3/(V1+V2) lower than 1 means preferably lower than 0.999 and preferably lower than 0.99 and such as for example 0.95 or lower.

In a preferred embodiment, the liquid flow is constant over time and the location of the amplification solution (liquid phase) in the flow channel and/or in the detection chamber is known by a time control of the liquid phase location in the flow channel. Preferably, the liquid phase location is known from a measure of a signal obtained in at least one location of the flow channel, from a gas/liquid phase transition, said measure being obtained by temperature shift, fluorescence signal change, electric signal, luminescence or light absorbance change.

### Amplification

In a particular embodiment of the method and device, at least one reagent necessary for polynucleotide molecule amplification is immobilized within the flow channel and/or the detection chamber such that when the solution is transported through the temperature regions, the reagents are brought into contact with the target polynucleotide molecule.

The amplification used in the method according to the invention is advantageously obtained by well known amplification protocols, including but not limited to the polymerase chain reaction (PCR), ligase chain reaction (LCR), Cycling probe technology (CPT), Nucleic Acid Sequence Based Amplification (NASBA), Strand displacement amplification (SDA), loop-mediated isothermal amplification (LAMP), Self-sustained sequence replication (3SR), Helicase-dependent amplification (HDA). The amplification methods are subdivided in two categories: thermocycling methods (PCR, LCR) and isothermal methods (CPT, NASBA, SDA, LAMP, 3SR, HDA). Also the application of variants of these amplification methods, e.g. reverse transcription PCR , real-time PCR, asymmetric PCR, or hot-start PCR are performed according to the methods of the present invention or in the devices/apparatus according to the present invention.

Preferably, the amplification is performed by PCR and the amplification solution comprises a DNA polymerase and primers substantially complementary to the target polynucleotide molecule.

In a preferred embodiment, the amplification is obtained by PCR comprising denaturation, annealing and elongation steps.

Preferably, prior to the step of submitting the solution to the amplification cycles, the solution is submitted to a denaturation step of more than 1 min, preferably more than 2 and even more than 4 min.

Preferably, an amplification cycle requires 4 different temperature steps: denaturation, annealing, hybridization and elongation.

In an alternative embodiment, an amplification cycle comprises 3 temperature steps: denaturation, annealing/hybridization and elongation. In this embodiment, the annealing step is performed at the same temperature as the hybridization step. Preferably, the annealing step is performed into the detection chamber and the annealing and hybridization steps are performed in the same temperature region.

In still a particular embodiment, an amplification cycle comprises 2 temperature steps: denaturation and annealing/hybridization/elongation. In this particular embodiment, the annealing and elongation steps are performed at the same temperature as the hybridization step. Preferably, the annealing and elongation steps are performed into the detection chamber and the annealing, elongation and hybridization steps are performed in the same temperature region.

Preferably, the reagents for polynucleotide molecule amplification comprise primer(s), dNTPs, a thermostable DNA polymerase and a buffer. Preferably, the primer(s) and/or the dNTPs comprise a fluorochrome labeled amplification precursor in order to form labeled targets.

Preferably, the (amplified) labeled target polynucleotide molecules are fluorescently labeled by incorporation of a fluorochrome labeled amplification precursor. Preferably, the fluorochrome of the amplification precursor comprises Alexa derivatives, aminocoumarin, CY3, CY5, CY7, Fluorescein, Rhodamine, Texas Red, Pacific Blue , Pacific Orange, Phycoerythrine derivatives, cyanine derivatives, acridine derivatives or coumarin derivatives.

In an alternative embodiment, the (amplified) labeled target polynucleotide molecules are fluorescently labeled by incorporation of intercalating dyes within the double helix, leading to fluorescence increase. In this particular embodiment, the reagents for polynucleotide molecule amplification comprise an intercalating fluorescent dye preferably selected from the group consisting of: SYBR Green, ethidium Bromide, acridine orange, SYTOX Blue.

In a preferred embodiment, the target polynucleotide molecule (nucleotide sequence specific of an organism or part of an organism) is a DNA nucleotide sequence.

In an alternative embodiment, the target polynucleotide molecule is an mRNA that is reverse transcribed into cDNA before the PCR.

In a specific embodiment, the method of the invention comprises the step of extracting from a biological organism or part of an organism a nucleotide sequence specific to that organism.

The buffer preferably comprises a salt composed of a cation and an anion, wherein the said anion has two carboxylic groups and one amine group, wherein the salt concentration in the composition is comprised between 10 mM and 400 mM and from 1% to 20% by weight of an exclusion agent. The anion is preferably glutamate.

Preferably, the reagents for polynucleotide molecule amplification comprise a hot start system. One particular method to obtain hot start system is by physical separation of at least one of the PCR reagent into the flow chip device. Alternatively, a hot start system is obtained by using a hot start Taq polymerase.

In another embodiment, at least one PCR cycle comprises the use of a thermostable DNA polymerase enzyme that is active at a concentration in salt comprised between 25 and 300 mM. The preferred salts are: potassium glutamate, potassium chloride and sodium chloride. The polymerase enzyme is preferably a *Thermus aquaticus* DNA polymerase enzyme. Thermostable means which still retains at least 50% of its initial activity after one PCR cycle. Active in salt (at a concentration in salt comprised between 25 and 300 mM) means an enzyme which shows preferably at least 5% and better at least 20% and still better at least 50% of its activity compared to its activity in solution with salt being lower than 25 mM.

In a preferred embodiment of this invention, the amplification is performed with tailed primers and the second primers being both present from the beginning of the amplification, without any destruction of the tailed primers. This method requires the use of an appropriate ratio between the tailed primers and the second primers, the former being at least 5 times lower than the latter, preferably 10 times lower.

The method of the invention is perfectly adapted for automated of multiplex real-time PCR reactions being performed on a micro-array. The number of targets to be detected is very large according to the number of immobilized capture molecules present on the micro-array.

In an embodiment, the micro-array is in contact with reagent for carrying out the amplification of one or more polynucleotide target molecule(s). In a preferred embodiment, between 1 and 4 target polynucleotide molecule(s), preferably between 1 and 10 target polynucleotide molecule(s) and more preferably between 1 and 20 target polynucleotide molecule(s) present in solution are amplified and detected and/or quantified in the same assay. In a further embodiment, preferably at least 2 or more, more preferably 3 or more, and even more preferable 4 or more target polynucleotides present in solution are amplified and/or quantified in the same assay. In another embodiment, between 20 and 1000 target polynucleotide molecules present in solution are amplified and detected and/or quantified in the same assay.

The present invention is also well adapted for the assay of DNA methylation in particular the cytosine-5 DNA methylation found in the Cp-G dinucleotides. In a particular assay the DNA is first treated with bisulfite for conversion of non methylate cytosine into uracil. Sequence discrimination is proceeded at the level of PCR amplification based on difference in the annealing of perfectly matched versus mismatched primers (see Eads et al 2000 Nucleic Acids Res 28, e33 incorporated for reference).. In another particular method, the discrimination is obtained at the level of the probe immobilized on the array using a probe matching a cytosine and another one matching a uracil. In this case the primers amplify both sequences and the discrimination is obtained different signal intensity on the 2 respective probes according to the presence of the matched or non matched amplicons.

The present invention is also particularly well adapted for discrimination of SNP into a DNA or RNA sequence. In a particular embodiment, the determination of the presence of a mutation or a deletion in an amplicon is based on the assay for the difference in the temperature of dissociation of the amplicons fixed either on the perfectly matched probe or on the non matched probe. Difference in the dissociation temperature is preferably obtained by gradual heating the hybridization chamber region and recording the signal of both the match and non matched probes. Difference in the rate of dissociation and/or the value of the signals according to the temperature will indicate the presence of the perfectly matched sequence compared to the mismatched sequence in the solution. Conclusion is best obtained if a difference of at least 4 and even better of 10 and even better 20 between the perfect match and mismatch signals is obtained at a given temperature.

### Micro-array

The micro-array according to the method and device is preferably a low or medium density micro-array having capture molecules immobilized in specifically localized areas of a solid support in the form of a micro-array comprising more than 4 capture molecules per cm², preferably more than 10, more than 20 and even more than 50.

The surface of a localized area is preferably comprised between 10 µm² and 1 mm² and preferably between 1 µm² and 100 µm².

The micro-array comprises preferably between 4 and 100000 capture molecules, preferably more than 10, and even more preferable more than 20. Preferably, the micro array contains about 1000 capture probes or less, more preferably 200 capture probes or less, like for example 12, 25, 40, 80 or 160.

The density of the micro-array is preferably comprised between 4 and 100000 spots per cm² and preferably between 10 and 1000 spots per cm², each spot being the localized area for one capture molecule. The amount of capture molecules immobilized on the support is preferably comprised between 20 and 2000 fmoles per cm².

The surface of the micro-array is preferably of 0.1 and 10 cm², better between 0.2 and 4 cm² and even more preferably between 0.5 and 2 cm².

In a preferred embodiment, the micro-array is a 2D-array or a line array.

The capture molecules have preferably a spacer portion and a capture portion. Only the capture portion of the capture molecule is specific of the amplicon. The capture molecule is immobilized to a solid support in such a way that the spacer portion is located between the solid support and the capture portion. Preferably, the capture portion of the capture molecule is separated from the surface of the solid support by a spacer portion of at least 6.8 nm long being preferably a sequence of at least 20 nucleotides and preferably more than 40 nucleotides long and more preferably at least 90 nucleotides and is comprise between about 20 and about 120 bases.

The spacer portion is better a nucleotide sequence, and the nucleotide at the distal end of the spacer portion is used to bind the capture molecule to the solid support. For this purpose the nucleotide at the distal end of the spacer portion is provided with an amino group, which forms a covalent bond with for example epoxy or aldehyde groups present at the surface of a pre-treated solid support.

The chemistry of immobilization of the capture molecules on the surface of the support has to be stable enough as to sustain the temperature stress required for the amplification. Means to control the rate of retention of the capture molecule on the support during or after the amplification process, is to incorporate in the micro-array positive detection controls being fluorescently labeled capture molecules.

In a particular embodiment, the micro-array comprises fluorescently labeled capture molecules (positive detection controls) which keep more than 50% of their fluorescence, preferably more than 80% and even better more than 90% at cycle 35 of the amplification as compared to cycle 1.

The capture portion of the capture molecule contains preferably from 10 to 100 nucleotides, preferably from 15 to 40 nucleotides, more preferably from 20 to 30 nucleotides specific of the amplicons produced during the PCR. Preferably, the capture portion of the capture molecule is comprised between 10 and 600 bases, preferably between 20 and 50 bases, more preferably between 15 and 40 bases.

In a specific embodiment, the capture molecules comprise a capture portion of 10 to 100 nucleotides that is complementary to a specific sequence of the amplicons such that said capture portion defines two non-complementary ends of the amplicons and a spacer portion having at least 20 nucleotides and wherein the two non-complementary ends of the amplicons comprise a spacer end and a non-spacer end, respectively, such that the spacer end is non-complementary to the spacer portion of the capture molecule, and said spacer end exceeds said non-spacer end by at least 50 bases.

In a preferred embodiment, the identification and/or quantification of the target polynucleotide molecule present in a sample is performed by monitoring the signal on the different locations of the micro-array with at least two measurements being done per location in at least two cycles of the amplification process. Subsequently the data are processed.

In another embodiment, the hybridization of an amplicon, specific of an organism, to the capture molecules forms a single spot signal at a predetermined location, whereby the detection of said single spot signal allows the discrimination of the specific amplicon from other homologous amplicons from other organisms.

### Labeling and detection

Hybridized targets are detected on the micro-array by a series of method described but not limited to the ones presented here under as long as they are compatible with the constraints given by the PCR. A non-labeled method has been proposed to be applicable on micro-array and is based on identification of the target by mass spectrometry adapted to micro-arrays (U.S. Pat. No. 5,821,060, which is hereby incorporated by reference herein in its entirety).

The label-associated detection methods are numerous. A review of the different labelling molecules is given in WO 97/27317, which is hereby incorporated by reference herein in its entirety. They are obtained using either already labeled primer, or by enzymatic incorporation of labeled nucleotides during the copy or amplification step or by intercalating agents followed by fluorescent detection (WO 97/27329, which is hereby incorporated by reference herein in its entirety).

The most frequently used labels are fluorochromes like Cy3, Cy5 and Cy7 suitable for analyzing a micro-array by using micro-array scanners (General Scanning, Genetic Microsystem). The resulting signal of target fixation on the micro-array is detected using fluorescent, colorimetric, diffusion, electroluminescent, bio- or chemiluminescent, magnetic, electric-like impedometric or voltametric (see e.g., U.S. Pat. No. 5,312,527, which is hereby incorporated by reference herein in its entirety), or radioactive detection.

The preferred labels are fluorochromes which are detected with high sensitivity with fluorescent detector. Fluorochromes include but are not limited to cyanine dyes (Cy3, Cy5 and Cy7) suitable for analyzing a micro-array by using commercially available micro-array scanners (as available from, for example, General Scanning, Genetic Microsystem). Preferably, the excitation wavelength for Cy3 is between about 540 and 558 nm with a peak at 550 nm, and the emission wavelength is between about 562 and 580 nm with a peak at 570 nm. Preferably, the excitation wavelength for Cy5 is between about 639 and 659 nm with a peak at 649 nm, and the emission wavelength is between about 665 and 685 nm with a peak at 670 nm. Preferably, the excitation wavelength for Cy7 is between about 733 and 753 nm with a peak at 743 nm, and the emission wavelength is between about 757 and 777 nm with a peak at 767 nm.

Fluorochromes are also possibly incorporated into the targets by chemical reaction such as the reaction of fluorescent dye bearing a N-hydroxysuccinimide (NHS) group with amines groups of the targets. Other useful fluorescent dyes in the present invention include cyanine dyes, fluorescein, texas red, rhodamine, green fluorescent protein. Patents teaching the use of such labels include U.S. Patent Nos. 3,817,837; 3,850,752; 3,939,350; 3,996,345; 4,277,437; 4,275,149; and 4,366,241.

In a preferred embodiment of the invention, the detection of the fluorescence signal related to the presence of the amplicons on the capture molecule takes party of a signal increase on the micro-array as compared to the fluorescence in solution. In a particular embodiment the difference of the detection of the fluorochrome present on the micro-array is based on the difference in the anisotropy of the fluorochrome being associated with a bound molecule hybridized on the capture molecule as a DNA double helix compared to the free moving molecule in solution. The anisotropy depends on the mobility and the lifetime of the fluorochromes to the detected. The method of assay for the anisotropy on micro-array is now available from Blueshift Biotechnologies Inc., 238 East Caribbean Drive, Sunnyvale, Calif. 94089 (http://www.blueshiftbiotech.com/dynamicfl.html).

A preferred detection method is based on the use of the forbidden angle for discrimination of the fluorescence emitted from the surface (bound targets) and the fluorescence present in the solution (free targets).

Preferably, the detection of the fluorescence emitted from the localized areas of the surface having hybridized target polynucleotide is assayed through an optically transparent solid support bearing the immobilized capture molecules in an observation angle which is within the forbidden angle.

In a preferred embodiment, the signal is detected within the forbidden angle at an observation angle θobin relative to the normal to the said surface, such that 90° > θobin > sin-1 (n2/nl), whereby the optically transparent solid support has a refractive index n1 and is in contact with the solution having refractive index n2, whereby n1 > n2.

Preferably, the observation angle is in the range of the critical angle plus 10°, preferably plus 5° and more preferably plus 3°.

Another particular detection method for discrimination of the fluorescence emitted from the surface (bound targets) and the fluorescence present in the solution (free targets) is the evanescent field.

In such particular embodiment, the detection of the fluorescence emitted from the localized areas is assayed after evanescence induced illumination of the surface having hybridized target polynucleotide.

The detection using an evanescent field is described for example in the US Patent Application 11/526,159. An "evanescent field" or "evanescent wave" is used herein with its commonly understood meaning, i.e., refers to an exponentially decaying electromagnetic field generated on the far or distal side of a totally internally reflecting interface that is illuminated by an incident light source. Generally, the excitation energy of the evanescent wave is the same as the energy of the wavelength of the incident light that was totally internally reflected. Typically, the evanescent field propagates with significant energy for only a relatively short distance from the distal surface of the interface (e.g., on the order of magnitude of its wavelength).

Preferably, the illumination is such as to obtain Total Internal reflection fluorescence (TIRF) and homogeneous evanescent field on the surface of the solid support having capture molecules immobilized thereon. Preferably, the excitation light source (1) produces an evanescent field. The evanescent field is preferably generated by an incident light source illuminating the surface of the solid support with an incidence angle comprised between about 60° and 90°. The evanescent field excites the label of the labeled amplicons and emitted signal is detected by a detector.

Another alternative detection method for discrimination of the fluorescence emitted from the surface (bound targets) and the fluorescence present in the solution (free targets) is based on illumination and/or detection focussed on the surface. In such embodiment, the detection of the fluorescence emitted from the localized areas is assayed after focalized illumination of the surface having hybridized target polynucleotide.

The excitation is preferably obtained by a laser beam which is focussed on the surface of the micro-array. Scanner method with a focusing of the laser beam used a confocal scanning method including a pin hole. Many such scanners are commercially available such as the PROSCANARRAY.RTM. line of scanners from PerkinElmer.RTM. Life, the Affymetrix 428 scanner, the Virtek Vision Chipreader line, etc. Some fluorescence laser based detection is now available for multiwell formats as for example the Safir from Tecan (Tecan Trading AG, Mannedorf, Switzerland; www.tecan.com). They could be adapted for the present invention.

In a particular embodiment, the detection of fluorochrome molecule is obtained preferably in a time-resolved manner. Fluorescent molecules have a fluorescent lifetime associated with the emission process. Typically lifetimes for small fluorochrome such as fluorescein and rhodamine are in the 2-10 nanosecond range. Time-resolved fluorescence (TRF) assays use a long-lived (>1000 ns) fluorochromes to discriminate assay signal from short-lived interference such as autofluorescence of the matrix or fluorescent samples which almost always have lifetimes much less than 10 ns. In one specific embodiment, the detection of fluorochrome is performed by using the PLEXOR.TM. Technology (Promega).

Preferably, the difference in the wavelength of fluorescence emission is obtained by fluorescence resonance energy transfer (FRET). In one specific embodiment, a primer is labeled with a fluorochrome (F1) having a given optimal fluorescent emission wavelength and serving as donor that is fluorescent when excited at its excitation wavelength in the solution.

In a preferred embodiment, the device for detecting a signal comprises a light source illuminating the sides of the insoluble solid support. The light source is preferably a non collimated laser source or a light emitting diode by a pair of optical fiber bundles as proposed by Aurora Photonics Inc. (26791 West Lakeview, Lake Barrington, USA; info@auroraphotonics.com).

Some fluorescent labels are of particular interest, such as nanocrystalline particles having fluorescent properties. The most common ones are the Quantum dots (Han et al., Nature Biotechnology, Vol. 19, p. 631, 2001). They are fluorescent and do not bleach with time or with illumination. Their stability makes them particularly suitable for the use in continuous reading, as proposed in this invention. Also, they contain metals that confer to these particles specific properties, so that other methods than fluorescence are also used to monitor their attachment to the capture molecules. Thermal heating of these particles is one of the parameters that is best monitored with time. The fact that the metal absorbs the energy of a light beam, preferably a laser beam, and induces heating of the particle, has been used as a basis for the detection of low density gold particles on a support, and even single particles are detected (Boyer et al., Science, Vol. 297, p. 1160, 2002). The method is called Photothermal Interference contrast.

Direct method for detection of the binding of the target molecules on capture molecule of the micro-array is the chemical cartography based on optical process of non-linear generation frequency spectroscopy (GFS) (L. Dreesen et al., Chem Phys Chem, Vol. 5, p. 1719, 2004). This technology allows the imaging in real-time of the vibrational properties of surfaces and interfaces with a submicron spatial resolution. The measurement is obtained by mixing at the surface of a substrate two laser beams, one having a fixed frequency in the visible (green) and the other having a variable frequency in infrared. The vibrational signature at the interface is obtained by measuring the light emitted by the sample in function of the frequency of the infrared laser beam. This method avoids labelling the target to be detected and so it represents a particular embodiment. Another technology for the direct measurement of nanoparticles is Rayleigh scattering. This method is based on the use of a light beam adapted in order to obtain an oscillation of the electrons in a metal particle so that an electromagnetic radiation is obtained from the particle, which are detected. (Stimpson et al., Proc. Natl. Acad. Sci. USA, Vol. 100, p. 11350, 2003) (real-time detection of DNA hybridization and melting on oligonucleotide micro-arrays by using optical wave guides) However until now the method is lacking the necessary sensitivity for application on biological samples.

Alternatively, Raman scattering and surface plasmon resonance are methods which particularly apply in the present invention, which techniques have been extensively used for the detection of antibody/antigen binding, but are also well suited for the multiparametric measurement of the micro-arrays and for the required sensitivity on biological samples. (Thiel et al., Analytical Chemistry, Vol. 69, p. 4948, 1997). In another embodiment, quartz crystal microbalances are applied, which are now sensitive enough that they measure changes of mass less than one nanogram (cf. Caruso et al., Analytical Chemistry, Vol. 69, p. 2043, 1997). This is one proposal for micro-array detection in real-time.. Cantilevers are another option for the detection of DNA on micro-arrays. (McKendry et al., Proc. Natl. Acad. Sci. USA, Vol. 99, p. 9783, 2002).

Also, another technology is the electrical detection of nanoparticles, which takes into account their metal properties. Electrochemical detection was first applied, but with low sensitivity. A more advanced and more sensitive method is the detection by differential pulse voltametry (Ozsoz et al., Analytical Chemistry, Vol. 75, p. 2181, 2003). The resistivity and the capacitance properties of the metal are among the best properties to be detected on electronic chips. The resistivity and the capacitance properties of the metal are also one of the best properties to be detected on electronic chips. The presence of a metal between two electrodes will induce a change of resistivity and of capacitance. The detection of the DNA or proteins is then observed when the capture molecules are present on one of the electrode (Moreno-Hagelsieb et al Sensors and Actuators B-Chemical, 98, 269-274, 2004). The capacitance assay of the gold labeled DNA has been described by Guiducci et al. ESSDERC 2002. Since electronic chips can be made of several plots, different targets are detected on different plots and the change in the resistivity or in the capacitance is recorded. If the methods have not yet been able to produce reliable and sensitive detections as required by the biological samples, it is, however, predicted that some of them will succeed to fulfil the requirements for the real-time detection. Another promising technology for measuring the binding of the target molecules on capture molecule of the micro-array is the chemical cartography based on optical process of non-linear generation frequency spectroscopy (GFS) (L. Dreesen et al. Chem Phys Chem, 5 , 1719-1725, 2004). This technology allows the imaging in real-time of the vibrational properties of surfaces and interfaces with a submicron spatial resolution. The measurement is obtained by mixing at the surface of a substrate two laser beams, one having a fixed frequency in the visible (green) and the other having a variable frequency in infrared. The vibrational signature at the interface is obtained by measuring the light emitted by the sample in function of the frequency of the infrared laser beam. This method allows avoiding labelling of the target in order to be detected.

In a preferred embodiment, the signal monitored on the different locations of the micro-array is selected from the group consisting of: colorimetry, fluorescence, time-resolved fluorescence, photothermal interference contrast, Rayleigh scattering, Raman scattering, surface plasmon resonance, change of mass, quartz crystal microbalances, cantilevers, differential pulse voltametry, chemical cartography by non linear generation frequency spectroscopy, optical change, resistivity, capacitance, anisotropy, refractive index and/or counting nanoparticles.

In an embodiment, some capture molecules are elongated by the polymerase and some others are in the same time hybridized with the amplified products which accumulate in solution during the thermal cycle. In one embodiment, the capture molecules elongated are detected during the temperature step of denaturation. In another embodiment, the capture molecules elongated and the labeled nucleotide molecules bound on their capture molecule are both detected during the temperature step of annealing and/or elongation.

In still another embodiment, the capture molecule bears both a fluorescent quencher or a fluorescent acceptor and a fluorescent donor. The quencher or the fluorescent acceptor is released due to a cut in the capture molecule and the fluorescent donor is detected and or quantified on the support. Preferably, such release of the quencher results from an enzymatic activity. Preferably the enzyme is specific of double stranded DNA or DNA-RNA and comprises but is not limited to enzymes having the activity of Uracyl DNA glycosylase (UNG), restriction enzyme, 3', 5' exonuclease, 5', 3' exonuclease, RNAseH. Preferably the enzyme is stable at 60°C and better at 80°C and still better at 94°C.

In a specific embodiment, the quenching effect on the capture molecule is obtained by a small distance between the quencher and the fluorescent donor. The capture molecule preferably comprises a hairpin or not as long as the quencher and the fluorescer are maintained at proximity. In a preferred embodiment, the quencher is 1.4 nm diameter gold nanoparticle and the fluorochrome is fluorescein, Rhodamine 6G, Texas red or Cy5. The quencher is preferably located at the free end of the capture probe and the fluorochrome is located at the end or close to the end of the capture molecule which is immobilized on the support.

The invention allows identification of the presence of a polymorphism by using a micro-array having different bounded single stranded capture polynucleotide sequences by the determination of a single signal resulting from the binding between the capture sequence and the target sequence, extending at least one nucleotide of the capture sequence beyond the 3' terminal nucleotide thereof in the 3' 5' direction using the target sequence as a template, said extension is effected in the presence of polymerisation agent and nucleotide precursor wherein at least one nucleotide incorporated into the extended capture sequence is a detectably-modified nucleotide;

One preferred support comprises polymer having chemical and thermal stability, low fluorescence and optical stability, preferably cyclo-olefin polymer such as Zeonex.RTM or Zeonor.RTM (Zeon Chemicals, Louisville, USA), or but not limited to, Topas, Udel, Radel or THV.

### Data analysis and quantification

### Analysis

In a preferred embodiment, the method is performed by monitoring the signal at different timings of the amplification on the different localized areas of the micro-array with at least 5 measurements being done per localized area in at least 5, preferably at least 10 different amplification cycles, more preferably at least 20 different amplification cycles. Subsequently the data are processed. In a specific embodiment, the process comprises more than 20 different amplification cycles, and/or no measurements are performed during the first 20 amplification cycles. In a preferred embodiment, the different timings of the amplification for measuring the fluorescent signal correspond to amplification cycles.

Preferably, the amplification cycles are determined by data analysis of a flow chip sensor. The sensor signal is analyzed in order to detect changes associated with the presence or movement or flow of liquid and preferably the detection of the transition phase within the flow and/or the passage of the front or end of the solution.

The sensor allows the counting of the cycle number by determination of the number of variation recorded by the sensor with time linked to the passage of liquid at the sensor location. Preferably, the number of transition phase of liquid to gas corresponds to the number of cycles. Counting the cycles allows providing a relationship between the spot values and the amplification cycles which is one requirement for real time amplification and/or quantification. Alternatively, and in the absence of a gas phase in the flow chip device, the amplification cycles are calculated from V1+V2 of the flow chip device features and flow rate. The cycle duration is the time for the solution to complete cycling through the flow channel and the detection chamber.

The duration of an amplification cycle is determined or calculated according to the features of the device V1+V2 (V1being the volume of the flow channel and V2 the volume of the detection chamber) which depend mainly on the flow channel section and its length inside the different temperature regions of the cartridge and the volume of the detection chamber (V2). The flow rate in the flow device relies on the speed of the pump or other system used for making the flow of liquid in the device. Determination of the flow rate and/or the time for performing one cycle in the given conditions of work is preferably done prior to the experiment as part of a standardization process.

In an alternative embodiment, the signal is monitored with time by performing at least two measurements per amplification cycle. In another embodiment, the amplification is obtained using at least 5 and better 10 and even better 20 amplification cycles, each comprising the three steps of denaturation, annealing and elongation, whereby each cycle is performed within 3 min, preferably within 2 and even within 1 min.

In another embodiment, measuring a fluorescent signal from the hybridized target polynucleotide molecules further comprises measuring a background signal for each of the localized areas and subtracting the background signal from the fluorescent signal for each of the localized areas. Preferably, the background signal is the local background around the localized area where the capture molecules are bound. Preferably the quantification of the spots and/or the data analysis is/are performed as described by de Longueville et al, 2002 (Biochem. Pharmacol. 64, 137-149).

In a preferred embodiment, the analysis the signal values is performed on a micro-array image having pixel values corrected by the pixel values of the image taken before the amplification or in one of the first ten amplification cycles (reference image). Preferably, the signal/background ratio is increased by a factor of 2, preferably of 5 and even of 10 after correction of the image. Advantageously, the background signal is lower than 500 and even lower than 200 on a 65000 grey level scale after correction of the image. Also as an advantage, the standard deviation of the pixels values within the local background of a spot is at least 2 times lower and even 5 times lower after correction of the image. The reference image is the image of the micro-array of a given device which includes all the reference spots like the detection controls or the hybridization controls but in which the spots related to the targets are negative and are considered as the blank. The target spots are negative before the starting of the experiment or in the few cycles where the amplification target concentration is still too low to give a signal to the spot. Preferably the first cycle(s) is the cycle 1 and still is one of the cycles until cycle 5 and still until cycle 10. In a particular embodiment, the reference is the result of data coming from several of the first cycles like the sum or the average of several images. The subtraction of the reference image to the test image allows to correct for the defects and most of the non specific signals originated from the device in a particular experiment. Gridding is preferably performed after correction of the image by an image from an earlier cycle. Preferably the position of the micro-array surface relative to the detector is kept constant for each of the image necessary for a given experiment. Also preferably the gridding adjustment of the image is performed only once and the same gridding parameters are used for each of the images taken for one experiment and in a particular device. The position of the device is then fixed compared to the position of the detector.

Still in a preferred embodiment, the analysis the signal values is performed on a micro-array wherein pixel values of the image of the micro-array from amplification cycle n is added to the pixel values of the image of the micro-array from amplification cycle n -1. The analysis is preferably performed on data originating from images of successive cycles. In a preferred embodiment, the different images values pixels are added and the resulting cumulative spot values are taken as a measurement for the appearance of a given target in the solution. In a preferred embodiment, cumulative spot values are plotted along the amplification cycles to detect and/or to quantify the target polynucleotide molecule(s) present in the sample. Preferably the cumulative spot values are plotted along the amplification cycles and/or use for the determination of the cut off (Ct) value and the quantification of the target.

### Quantification of the target

Quantification has to take into account not only the hybridization yield and detection scale on the micro-array (which is identical for target and reference sequences) but also the extraction, the amplification (or copying) and the labelling steps.

In a preferred embodiment, the quantification of the target polynucleotide molecule(s) present in the sample is obtained by comparing the signal values (of the different locations) with a fixed value.

In another embodiment, the quantification of the target polynucleotide molecule(s) present in the sample is obtained by comparing the number of amplification cycles necessary to reach a fixed value (CT) with the CT of a reference polynucleotide molecule, which is preferably a polynucleotide molecule amplified in the same solution and detected on the same micro-array as the target polynucleotide molecule. Alternatively, the quantification of the target polynucleotide molecule(s) present in the sample is obtained by comparing the number of amplification cycles necessary to reach a fixed value (CT) with a standard curve wherein CT values are plotted against standard concentrations.

In another alternative embodiment, the quantification of the target polynucleotide molecule(s) present in the sample is obtained by comparing the kinetic constant of the signals of at least two amplification cycles.

In another embodiment, the quantification of the target polynucleotide molecule(s) present in the sample in copy number is obtained by comparing signal values of the target polynucleotide molecule(s) to the signal values obtained for a predetermined number of standard polynucleotide molecules added to the amplification solution at known copy number. Advantageously, the standard polynucleotide molecule is added to the initial biological sample, or after the extraction step, and is amplified or copied with the same primers and/or has a length and a GC content identical to, or differing by no more than 20% from the target polynucleotide molecule.

Preferably, the hybridization yield of the standard polynucleotide molecule (on its specific capture molecule) is identical to, or differ no more than 20% from, the hybridization yield of the target polynucleotide molecule. Specifically, the standard polynucleotide molecule is designed as a competitive internal standard having the characteristics of the internal standard found in the document WO 98/11253, which is hereby incorporated by reference herein in its entirety. Quantification based on the use of such standard is also described in WO 98/11253.

Said standard polynucleotide molecule, external and/or internal standard, is also advantageously included in the kit according to the invention, possibly with all the media and means necessary for performing the different steps according to the invention (hybridization and culture media, polymerase and other enzymes, standard sequence(s), labelling molecule(s), etc.).

### Device and Kit

The process according to the invention is preferably performed by using a specific identification (diagnostic and/or quantification) kit of a biological organism or part of an organism comprising means and media for performing the method of the invention. Specifically, a preferred kit comprises: a flow chip device according to the method of the invention, reagents for amplification including primer(s), dNTPs, a DNA polymerase and a buffer. The primer(s) and/or the dNTP include a fluorochrome labeled amplification precursor in order to label the amplified product.

In a particular preferred embodiment, the device comprises 4 regions being separated to be heated independently. One region is dedicated for denaturation step of PCR with temperature between 85 and 100°C preferably 95°C to 99°C, a second region is dedicated to annealing step at a temperature between 37°C and 70°C preferably between 50°C and 65°C and a third region is used for elongation step at a temperature between 60°C and 80°C preferably between 68°C and 75°C. The 4^{th} region is a region that is heated at any kind of temperature to modulate the size of one of the 3 other regions. A thin flow channel is passing through the 4 regions of the device, this channel having a section of 1 mm² preferably 0.25 mm² and a specific length in each region. The total volume of the flow channel is between 2 mL and 20 µL preferably between 300 µL and 100 µL. Preferably, the size of the channel in the first region is between about 5-40%, preferably between 10-30% like for example about 25% of the total size of the flow channel, in the second region between about 10-60%, preferably 20-50%, like for example about 35%, in the third region between about 10-50%, preferably about 15-40%, like for example about 25% and in the fourth region between 0% to about 40%, preferably between 0% and 30%, like for example about 15%. The second region also contains a detection chamber connected to the channel. This detection chamber contains a spotted micro-array.

Preferably, the substrate is which the flow channel is disposed comprises an opaque polymer. Preferably, all the sides of the detection chamber are black or coloured in black except the surfaces crossed by the illumination and by the emission light.

In a particular embodiment, the surface of the flow chip device in contact with the solution is in cycloolefin or elastomeric polymer. Cycloolefin polymer is preferably selected from Zeonex.RTM or Zeonor.RTM (Zeon Chemicals, Louisville, USA), Topas, Udel, Radel or THV.

In still another particular embodiment, the surface of the temperature region comprising the detection chamber is at least 40% of the total surfaces of the temperature regions.

In a preferred embodiment of the kit, the length of the specific sequence of the capture portion is comprised between about 10 and 600 bases, preferably between 15 and 50 bases, more preferably between 15 and 40 bases. In a preferred embodiment of the kit, the specific sequence of the capture molecule (capture portion), able to hybridize with their corresponding target nucleotide sequence, has a sequence having between 15 and 50 bases separated from the surface of the solid support by a spacer portion of at least 6.8 nm. In another embodiment of the kit, the spacer portion is a nucleotide sequence of more than 20 bases, preferably more than 40 bases, more preferably more than 90 bases.

Concentrations of capture molecules between about 600 and about 3,000 nM in the spotting solutions are preferred. However, concentrations as low as about 100 nM still give positive results in favourable cases (when the yield of covalent fixation is high or when the target to be detected is single stranded and present in high concentrations). Such low spotting concentrations correspond to a density of capture molecules as low as 20 fmoles per cm². On the other hand, higher density was only limited in the assays by the concentrations of the capture solutions. Concentrations higher than 3,000 nM give good results.

In the kit according to the invention, the capture molecules are present on the insoluble solid support in localized areas having a surface area of between 1 µm² and 75 mm² and preferably between 0.005 and 0.2 mm².

In a preferred embodiment, the micro-array contains at least 20 and preferably 50 and even more than 100 localized are per cm². Preferably, the micro array will contain about 1000 localized areas or less. The micro-array covers a surface of at least 0.1 and better 1 and even better at least 4 cm² of the solid support. Generally, the micro array will cover a surface area of about 10 cm² or less.

In a particular embodiment, the support and/or the chamber material is selected from the group consisting of glass, an electronic device, a silicon support, a plastic support, silica, metal and a mixture thereof, wherein said support is prepared in a prepared in a format selected from the group consisting of slides, discs, gel layers and microbeads. In still a specific embodiment, the support and/or the chamber material comprise cycloolefin polymer preferably Zeonex.RTM or Zeonor.RTM (Zeon Chemicals, Louisville, USA), Topas, Udel, Radel or THV.

The flow chip device is preferably a disposable device. The temperature controllers and adjusters, e.g. the heaters and/or coolers are preferably not part of such a device, particularly of a disposable device. In alternative embodiments heaters and/or coolers are part of the device. In particular embodiment, the device comprises additional compartments, e.g. for preparation of the amplification solution (i.e. preparation of the sample).

Also, the fluid transportation system or parts of the transportation system, e.g. the pump is preferably not part of a disposable device. In some embodiments, the fluid transportation system is comprised within the device. The structure or pattern of the temperature regions of the detection chamber is preferably reflected in the structure or pattern of the heaters and/or coolers of the temperature control and adjustment system. Also the device has preferably patterns of heat conducting and/or insulating surfaces.

### Apparatus

In a preferred embodiment, the apparatus for performing the method of the invention comprise system operatively disposed to transport fluid through the flow channel and to make the solution cycling inside the device. Preferably, this system comprises a pump, a compressing element magnetic beads and the like or combinations thereof.

In a still preferred embodiment, the rate of liquid flow in the flow channel is comprised between 6 µL/min and 3000 µL/min and preferably between 50 and 500 µL/min.

In a preferred embodiment, the apparatus comprise a flow chip sensor being preferably a heat detector, a fluorescence detector or a light absorbance detector.

The flow chip sensor is preferably a sensor which is fixed upon the flow device and which allows to record a physical parameter including but not limited to temperature, optical density, light scattering, fluorescence, refractive index, and electrical resistance.

The sensor signal is analyzed by the apparatus in order to detect changes associated with the presence or movement of liquid and preferably the detection of the transition phase within the flow and/or the passage of the head of the solution. Timing for the presence of the liquid in the detection chamber is preferably deduced from the analysis of different parameters including the position of the sensor on the flow chip, the timing of the signal change and the flow rate of the liquid. The volume of the liquid is used in order to determine the duration of the liquid present into the detection chamber. Example of velocity determination of the flow is presented in GB2433259 using the absorbance of the DNA under illumination of the flow channel with UV light. The number of cycles is preferably obtained from the counting of the number of variations in the sensor recording with time linked to the passage of liquid at the sensor location.

In still a particular embodiment, the position of the liquid in the flow device is calculated from the timing of the assay and the rate of the liquid flow in the device. The rate of the flux is corrected for the temperature of the region for the calculation of the flow position in the device according to the time and the distance.

In a preferred embodiment, the different temperature regions of the flow chip device are separated from each other by a double separation preferably including an air layer.

The heating system is preferably selected from the group consisting of a Peltier device, a resistive heater, a heat exchanger and an indium thin oxide element. The different regions of the amplification steps are preferably surrounded by the heating process located on both side of the regions one being below and the other one above the flow chip regions. The detection chamber is best heated by one side of the chamber, the other one being open for illumination of the micro-array. In a particular embodiment, the region of the annealing is the same as the detection chamber and one of the heater elements covering the annealing region is cut in a way as to let the illumination beam to reach the micro-array for detection. In a particular embodiment, a single temperature controller regulates the temperature of all the temperature regions. In an alternative embodiment, the temperature controller is one of a plurality of temperature controllers, each temperature controller separately regulating the temperature of one temperature region. The temperature controller is capable of regulating the temperature within the at least 2 different temperature regions to obtain a PCR comprising denaturation, annealing and elongation steps.

A heating system is preferably composed in its simplest version of the following relevant components: a thermocouple, a transmitter, a converter and a heater. The heating system aims to generate substantially constant temperature at each temperature region of the device allowing accurate amplification and/or hybridization. The generated temperature in each temperature region is kept substantially constant during the amplification process. The thermocouple, sticks as close as possible of the region to be heated, and measures the temperature thought the transmitter. This temperature information is given to a computer via the converter. The software compares the real temperature measured to the temperature set point requested by the final user at regular interval, such as every second. If the measured temperature is higher than the requested one, the heater is simply stopped. If the measured temperature is lower than the set point, the system continues the heating process.

Preferably the heating and cooling systems allow temperature variation of less than 1°C and more preferably of less than 0.25°C. Preferably, the uniformity of heat on the surface of the detection chamber is at least 1°C and preferably at least 0.1°C. Preferably the accuracy of the temperature is better than 0.5°C and preferably better than 0.1°C.

Besides the heating system, the detection requires an illumination light source and a detector for measuring a signal from the bound labeled target polynucleotide molecule. Preferably, a light source generates a beam of light to excite the bound labeled targets bound at the flat surface of the reaction chamber. The detection has to be settled in such a way as to obtain the same detection efficiency on the overall surface covered by the micro-array to be analyzed. A typical detector used in this context is a CCD camera capable to take a picture of the whole micro-array. In a preferred another embodiment, the resolution of the optical system is between 0.1 microns and 500 microns and more preferably between 10 and 100 microns.

In a preferred embodiment, the excitation light is a (red) laser diode preferably having a wavelength of 635 nm ± 5 nm. The laser diode is for example a Pearl™ 2W 639nm operating at temperature comprised between 10 and 35°C and having an output power of 10 to 2000 mW. The laser has preferably a low heat emission component in order to reduce distortion. Preferably, illumination comes from below the micro-array. The laser beam is also preferably perpendicular (+/- 10 °) to the flat surface having fixed capture molecules. A mirror is also preferably placed between the laser beam and the flat surface to reduce linear beam length. Preferably, the spectral line width is maximum 5 nm and even 3 nm in order to avoid nonspecific excitation and emission overlap. Preferably, the beam homogeneously illuminates the total surface of the micro-array, being preferably a surface of for example 20 x 10 mm. Homogeneity of illumination should be at least 95% over the whole surface and even at least 98%. In still another embodiment, the laser scans the micro-array surface.

Near Gaussian profiles are obtained by spatial filters or coupling to single mode optical fibers or alternatively by using solid state lasers such as DPSS. Preferably, the laser beam generated by the light source is nearly collimated and nearly Gaussian. In a preferred embodiment, an optical element is used to homogenise the illumination intensity over the entire micro-array surface such as refractive or diffusive optical elements. An exchangeable excitation filter is used to collect only the wavelengths of interest. An additional filter wheel is preferably placed and used as an attenuation filter to precisely regulate the laser power. This filter wheel is shaded differently at variable known absorption levels. A lens that is anti-reflection coated is used for focusing the laser beam on the flat surface of the reaction chamber. In a preferred embodiment, the detector comprises an optic lens. Emitted light is focused through an optical lens to a detector for detecting the number of photons present therein. The detector is preferably a cooled CCD camera. The camera is preferably a monochrome CCD. The camera is selected in order to provide maximum Quantum Efficiency at the wavelength of emission which is preferably 670 nm. The CCD quantum efficiency is preferably at least 20 % at 670 nm, preferably at least 50%. Preferably the pixel size lies between 8 and 24 µm. The CCD sensor area is at least 20 x 10 mm in order to obtain a 1:1 ratio and best image perspective. The CCD camera is preferably a full frame camera. Image digitization is preferably at least 12 bits in order to obtain sufficient quantification levels and better at least 14 bits. The exposure time is preferably at least 1 sec and preferably shorter than 10 sec and still preferably less than 5 sec and better less than 3 sec to allow real-time quantification. Mirrors are preferably avoided to prevent dust accumulation and picture deterioration. The lens has preferably the following features: a large image circle (i.e. a diameter of 43.2mm), a large aperture: f/2.8 going down to f/16, a short focal length (i.e.40 mm), a macro capability of less than 10 cm, is suitable for a 1:1 size ratio. In a specific embodiment, an emission filter that transmits light having a wavelength greater than about 665 nm is added. The emission filter is preferably placed on the lens or close to the lens. The emission filter has preferably the following features: a band pass with a low frequency pass, a cut-on frequency of 665 nm and cut-off at 700 nm, a transmittance of bandwidth higher than 0.8 T and a transmittance outside of bandwidth lower than 0.001 T, a transmittance between 500 and 665 nm lower than 0.0001 T, an accuracy of bandwidth ± 3 nm. In a preferred embodiment, the illumination light source produces an excitation light which is directly focused on the flat surface of the reaction chamber, wherein the excitation light reaches the micro-array surface within an angle comprised between 45 and 135°. Preferably, the excitation light reaches the surface of the support with an angle of about 90°, thus vertically. Calculated according to the normal, this angle would be about 0°. Thus, the excitation light reaches the flat surface of the reaction chamber within an angle which does not induce internal reflection of light as provided by evanescence wave.

In a preferred embodiment, the detection is performed using the forbidden angle method as described in the WO 2009/013220. The flow chip device (being placed in the apparatus) comprises the capture molecules are immobilized on the surface of an optically transparent solid support having a refractive index higher than 1.30 and a thickness of at least 0.5 mm and preferably at least 3 mm, wherein said solid support has two surfaces (S2 and S3) inclined relative to the surface (S1) of the support on which the capture molecules are immobilized, one (S2) being optically transparent and used for collecting light emitted from the localized areas of capture molecules in the forbidden angle (θobin) and inclined by an angle of between 90 and 60° compared to the solid support surface and the other one (S3) opposite being black or covered with a colour being black or covered with a colour having an absorption corresponding to the wavelength of the emitted light and wherein the device is positioned onto the apparatus in order for the light emitted in the forbidden angle (θobin) through the inclined surface S2 to reach the detector. Preferably, the observation angle is in the range of the critical angle plus 10°, preferably plus 5° and more preferably plus 3°. The camera is preferably mounted at a fixed observation angle θobout relative to the normal to the side surface of the support, such that 0° > θobout >θc out with θc out = Arcsin (n1 /n3 cos(θc)). With n1 being the refractive index of the optical block and n2 is the refractive index of the solution preferably a water solution (n2∼1.33) and n3 the refractive index of the air. In another embodiment, the signal is a scattered light from the bound labeled target polynucleotide molecule in response to illumination. In an alternative embodiment, the signal is the result of light diffraction from the bound labeled target polynucleotide molecule in response to illumination.

In a preferred embodiment, the incident light source, the solid support and the detector are not moving relative to each other during the detection. This is the simplest system. The CCD camera collects the light emitted from the solid support surface in a single acquisition.

The processes of heating for amplification and detection are performed in the integrated system. In a particular instrument described in example 5, the light source is directed on the surface of the support opposite to the surface in contact with the thermostatized heater.

The present invention also covers the machine and apparatus necessary for performing the various steps of the process mainly for diagnostic and/or quantification of a (micro)organism or part of an organism possibly present in a sample that comprises: a) capture molecules bound to an insoluble solid support surface at specific locations according to a micro-array; b) a device for thermal regulation; c) a device for detecting a signal formed at the location of the binding between an amplicon and a capture molecule; and d) a computer program for transforming the signal into digital data.

In a preferred embodiment the apparatus also comprises:
- a storage system for storing the data of the different measurements,
- a controller repeating the steps of illumination, detection and storage,
- a data analysis of the flow chip sensor to determine the liquid position in the flow chip device,
- a program for processing the data in order to detect and/or quantify the amount of polynucleotide molecule present in the solution before the amplification.

In another embodiment, the computer program further recognizes the locations of the micro-array where a signal is formed.

In the apparatus, the capture molecules are preferably single-stranded capture molecules being covalently bound in a location of a micro-array to an insoluble solid support, wherein said capture molecules comprise a capture portion of between 10 and 600 bases, said capture portion being able to specifically bind to said amplicon.

The apparatus preferably has a detector selected from the method group consisting of: colorimetry, fluorescence, time-resolved fluorescence, photothermal interference contrast, Rayleigh scattering, Raman scattering, surface plasmon resonance, change of mass, quartz crystal microbalances, cantilevers, differential pulse voltametry, chemical cartography by non linear generation frequency spectroscopy, optical change, resistivity, capacitance, anisotropy, refractive index and/or counting nanoparticles.

In a particular embodiment, the fluorescent scanner uses a laser beam including a confocal scanning method and also preferably a pin hole.

In a particular embodiment, the apparatus further comprises: a storage system for storing data from different measurements for at least 5 different locations of the support at a defined timing of a thermal cycle; a controller repeating the steps of detection and storage at least one time in at least one thermal cycle for each location of micro-array; and/or a computer program for processing the data obtained in at least one thermal cycle in order to detect and/or quantify the amount of nucleotide molecule present in a sample before amplification.

In a particular embodiment, the apparatus further comprises: a laser source; a focusing device for a laser beam produced by said laser source; a photomultiplier and a pin hole.

In another embodiment, the apparatus further comprises a computer program for converting a signal formed at a location into data associated with the presence of a particular target.

In a specific embodiment the apparatus is a multifunctional apparatus for amplification and detection of genes, DNA and polynucleotide sequences which performs PCR amplification, polynucleotide detection; real-time PCR, quantification real-time PCR, micro-array detection and/or quantification and SNP detection.

The flowchart of figure 9 describes an embodiment in which the real-time PCR apparatus is controlled by a programmable computer. The detector can be a sensitive CCD camera to take pictures of the micro-array while illuminated by the laser. Reference is made to the example 7 and figure 10.

At STEP 1, the user is prompted to fill in the required parameters, such as:
- time exposure of the CCD camera,
- laser power of the light source,
- denaturation temperature,
- annealing/hybridization temperature,
- elongation temperature;
- speed of the pump,
- detection (or image acquisition) in liquid or gas phase,
- the duration of an amplification cycle or the determination of an amplification cycle as counted by a sensor ,
- the moment an image of the micro-array is acquired by the detector.

### Explanation of the different parameters:

The "time exposure" of the CCD camera corresponds to the time for taking a picture of the micro-array in the detection chamber. A time of exposure of 5 sec is used in example 7.

The "laser power" defines the power of the light source for illumination of the micro-array in the detection chamber. In example 7, a high intensity laser of 2W is used.

The three temperatures of the PCR amplification are set in the following manner.

### 1. Denaturation temperature

In the instrument, the temperature of the first heating block (upper and lower) is set to a fixed value corresponding to denaturation step of PCR. As a result, the temperature region of denaturation of the cartridge is heated to the "denaturation temperature". The denaturation temperature is comprised between 85 and 100°C preferably 95°C to 99°C. In example 7, 95°C is used as denaturation temperature.

### 2. Annealing/hybridization temperature

In the instrument, the temperature of the second heating block (upper and lower) is set to a fixed value corresponding to annealing/hybridization step of PCR. As a result, the temperature region of annealing/hybridization of the cartridge is heated to the "annealing/hybridization temperature". The annealing/hybridization temperature is comprised between 37°C and 70°C preferably between 50°C and 65°C. In example 7, 58°C is used as annealing/hybridization temperature.

### 3. Elongation temperature

In the instrument, the temperature of the third heating block (upper and lower) is set to a fixed value corresponding to elongation step of PCR. As a result, the temperature region of elongation of the cartridge is heated to the "elongation temperature". The elongation temperature is comprised between 60°C and 80°C preferably between 68°C and 75°C. In example 7, 72°C is used as elongation temperature.

Each heating block is thermally regulated by a thermocouple placed inside the aluminium elements. An external PID regulating electronic device (OMRON) sent power to the heating resistance at regular interval to maintain the desired temperature.

Air pulse radiators were connected to each heating aluminium blocks to allow them to go down in temperature in a reasonable time when needed.

Optionally, additional temperature region is incorporated in the device that is heated at a given temperature to modulate the size of one of the 3 other regions.

The "speed of the pump" is one of the key parameters defining the flow rate in the cartridge. In example 7, the pump speed is set to 13 rpm.

The channel section, its length inside the different temperature regions of the cartridge (at which the temperature is regulated) and the volume of the detection chamber (V2) allows the calculation of the device volume (V1 + V2). The speed of the pump defines the flow rate and thus the duration of each of the amplification step (denaturation, annealing/hybridization and elongation).

For example the integrated flow chip device provided in figure 4 which has the dimension of a credit card (80 mm x 50 mm x 2 mm), comprises a flow channel (channel section: 500 µm x 500 µm) inside 4 different temperature regions (Region 1: channel of 20 cm long; Region 2: channel of 40 cm long; Region 3: 15 cm long and Region 4: 20 cm long) and an interconnected detection chamber in region 2. For a pump speed of 13 rpm, the duration of a complete cycle through the four temperature regions takes about 90 sec. The duration of one cycle is preferably predetermined by standardisation or calibration of the system using the given parameters of work.

Depending on the volume of solution (V3) introduced into the cartridge compared to the total volume of the device (V1 + V2), there will be two phases in the cartridge (gas/liquid) or only one phase (liquid).

The "presence of a gas phase" means that the volume of solution (V3) introduced in the flow chip device is lower than the total capacity volume of the device (V1 + V2).

The detection is performed in liquid or gas phase.

The presence of a gas phase in the device, make it possible to determine "the number of amplification cycles" (comprising denaturation, annealing/hybridization and elongation) experimentally. Counting the cycles is for example performed by a thermocouple probe which detects the passage from gas to liquid phase at a specific location of the flow channel. By knowing the speed of the pump and the timing when the solution reaches the section of the channel controlled by probe, it is possible to perform the image acquisition of the micro-array either in liquid or in gas phase.

Alternatively to the counting of the cycle with a probe, it is also possible to calculate the "duration of a cycle" according to features of the flow chip device such as the flow channel section, its length inside the different temperature regions of the cartridge (at which the temperature is regulated) and the speed of the pump.

The "moment the micro-array is detected" by the CCD camera defines when the users want to acquire an image of the array. It is either every cycle (one time or several times) or at predetermined cycle numbers as counted by the sensor or at predetermined intervals corresponding to the cycle duration.

In example 7, the image acquisition is performed at cycle 2, 5, 10, 12, 14, 16, 18, 20, 21, 22, 23, 24, 25, 26, 27, 28, 29, 30, 31, 32, 33, 34, 35, 36, 37, 38, 39 and 40 as determined by the sensor when the detection chamber was in gas phase. This parameter defines the number of image to be acquired by the detector during the amplification.

At STEP 2, the system is initialized: the essential parameters of the instrument are checked: temperature of the heating blocks, laser power of the illumination source, temperature of the CCD camera, the sensor and speed of the pump.

At STEP 3 happens the heating of the 1^{st} heating block in the instrument to the predefined denaturation temperature. In the flow chip device, the temperature region of denaturation is heated to the denaturation temperature. The same happens for the annealing/hybridization and elongation temperatures.

At STEP 4, the liquid is flowed through the denaturation region of the flow chip device and denaturation takes place. Optionally, the speed of the pump is reduced to maximize the denaturation time of the first amplification cycle.

At STEP 5a, the liquid is flowed through the annealing/hybridization region. Annealing of the primers takes place as well as the hybridization of target amplicon on the micro-array.

At STEP 5b, optionally according to the program, an image of the micro-array is acquired by the detector at a given amplification cycle, the acquired images are gathered for quantification and extraction of the signal and background values for each target present on the support and cycle wise data analysis is performed.

At STEP 6, the liquid is flowed through the elongation region to perform the elongation step.

At STEP 7, the determination of the amplification cycle occurs. The amplification cycle is either determined experimentally by counting the cycles with a sensor (e.g. a probe thermocouple). The sensor data is recorded and data analysed for determining the cycle number.

Alternatively, the duration of an amplification cycle is determined theoretically according to the flow device volume including the detection chamber and the speed of the pump. The cycle duration is the time for the solution to complete cycling through the flow channel and the detection chamber. In still another mode of calculation, the amplification cycle time is known and is part of the system parameters

If the number of image acquisition to be done is not reached, then the program cycles back to STEP 4.

If the number of image acquisition to be done is reached, the STEP 8 occurs: Data analysis is completed by an overall review of results and data analysis including reporting of the results. Data analysis includes the treatment of the array surface image for determination of the spot location and the determination of a value associated with the spot for a given target.

Quantification also preferably includes various corrections and determination of a Ct value and the quantification of the target present in the starting solution. Different methods for array analysis are known as well as the way to treat the data in real time PCR for quantification of the target (see de Longueville et al 2002 Biochem. Pharmacol., 64, 137-149; Meneses-Lorente et al 2003 Chem. Res. Toxicol., 16, 1070-1077; Hamels et al 2001 Biotechniques, 31, 1364-1372 ; WO 02/097135 and US 2004/0161767 introduced herein as references).

The present invention is further illustrated by the following non-limiting examples.

### Examples

### Example 1: real-time simplex PCR detected onto a micro-array into a flow chip device

A first flow chip device was prepared by the combination of disposable plastic cartridges, flexible tubing (ref: C-flex 06422-2; Cole-Palmer, Vernon hills, IL, USA), pump tubing (ref: 049ef0a-051, Watson-Marlow, Stockholm, Sweden) and detection chamber in order to generate a closed channel loop.

Disposable plastic cartridges were made of 2 pieces of Topas plastic moulded and welded to generate a channel with a section of 1 mm². There were 3 different disposable plastic cartridges (long, medium, small) with respectively 3 different lengths of channel: 30 cm (long), 20 cm (medium) and 10 cm (small). The disposable plastic cartridges had an inner and an outer port that could be connected to flexible tubing.

The detection chamber had an elliptic shape and was 7 mm (width) x 20 mm (length) x 100 µm (height). The detection chamber was closed on the upper side by a black plastic enclosure 1.5 mm thick and on the bottom side by an optical block 3.5 mm thick. The detection chamber was designed to hold about 10 µL of solution. The optical block was in Zeonex of optical grade. The surface was perfectly smooth and flat avoiding scratches and dusts. The optical block was activated by plasma treatment for production of epoxy groups on the surface (P2i, Abingdon, UK). The capture nucleotide sequences were printed onto the optical block surface with a home made robotic device using 250 µm diameter pins. The spots had 400 µm in diameter and the volume dispensed was about 0.3 nL. Optical blocks were dried at room temperature, laser welded on the detection chamber, and stored at 20°C until used. The side part opposite to the observation side of the optical block was covered with a black painting.

The flow chip device was prepared as follows.

It contained 4 regions:
Region A was made with 15 cm of flexible tubing attached onto the inner port of 1 medium plastic cartridge.
Region B was made with 10 cm of flexible tubing fixed on one end onto the outer port of the region A plastic cartridge and on the other end onto the inner port of the detection chamber. The outer port of the detection chamber was fixed with a flexible tubing of 5 cm, then 1 long plastic cartridge followed by 5 cm of flexible tubing.
Region C was made with 22 cm of flexible tubing.
Region D was made of a pump tubing (#049.EF0A.051, Watson-Marlow, Stockholm, Sweden) that was connected to the end of the flexible tubing of region C and to the beginning of the flexible tubing of region A making a closed loop.

The flow chip device could be opened at the junction of region D and A allowing the introduction of a solution into the flow device. Once the liquid was into the flow chip device, it was closed and the pump tubing was introduced into the peristaltic pump (#Serie 100, Watson-Marlow, Stockholm, Sweden) so the liquid could flow in the closed loop flow chip device. The total volume capacity of the flow chip device including the flow channel and the detection chamber was 2 mL. The flow chip device was placed inside a flow instrument. This instrument was made of 3 aluminium zones that were heated independently at different temperature (99°C, 58°C and 72°C). Region A of flow chip device was placed in the aluminium zone heated at 99°C, Region B in the zone at 58°C and Region C in the zone at 72°C. The thermal contact between the flow chip device and the aluminium blocks was made by using a layer of heat sink compound (Dow Coming 340, Dow Coming GMBH, Wiesbaden, Germany) between the aluminium surface and flow chip device bottom surface. The detection chamber was placed exactly over the laser and in front of the camera in order to make pictures of the micro-array in the detection chamber using the forbidden angle. On the top of the 3 heating zones, 3 aluminium covers were placed in order to keep the heat around the flow chip device.

The micro-array that was spotted on the optical block of the detection chamber was 9 X 19 spots of 400 µm and contained spots of Cy5 labeled detection control 5' end amino-polynucleotides: Detection control: (SEQ ID NO. 1)

5'NH2-TACCTACTACGCTACACGAACCTACAAGACAAGATAAAGACAGACTCATG-
3'Cy5.

This labeled capture probe was spotted at a concentration of 500 nM.

The micro-array also contained unlabeled capture molecules specific for different sequences and one of these capture being specific for the target GUT comprising a capture portion and a spacer portion (underlined):
GUT capture probe: (SEQ ID NO. 2)

5'NH2-
TTATTCACAACATTTCGATTTTTGCAACTACTTCAGTTCACTCCAAATTAGGGACTGG
CTGCTATTGGGCGAA-3'

The unlabeled capture probes were spotted at a concentration of 12 gM.

After spotting, plastic optical blocks were placed in an oven 30 min at 20°C under humidity and then at 60°C for 30 min under humidity. Plastic optical blocks were then washed 1x5 5 min in SSC2X pH 7 + BSA 1% + SDS 0.1%, 2 x 1 min in H₂O and finally 1 x 3 min in boiling water. After the washing steps, plastic optical blocks were dried at room temperature and welded onto the bottom of the detection chamber to generate a closed detection chamber containing the micro-array.

A PCR mix was prepared to amplify specifically a target GUT with one primer pair being labeled with Oyster at 5'end.

500 µL of a PCR mix containing the PCR Buffer 1X (Taps 50 mM, Tris-HCL 95 mM, MgCl₂ 2 mM), BSA 0.05%, a couple of primers (5'Oyster-GGGACCCTCGCCCAGAAAC-3' (SEQ ID NO. 3) and 5'-CCACCTGCTGACCCCGTC-3' (SEQ ID NO. 4)) each at 1 gM, Supersalt Taq Polymerase 1U/50 gL, 200 µM ofdATP, 200 pM of dCTP, 200 pM of dGTP, 100 pM of dTTP and 300 µM of dUTP and 100 copies of GUT target DNA were prepared. The PCR buffer IX also contained glutamate and dextran as provided in WO2009/086608.

400 pL of this PCR mix were incubated at 99°C for 5 min before entering the flow chip device inside a flow instrument and were processed with this program: 40 cycles of 1 min 20 sec with a denaturation step at 99°C, annealing step at 58°C and elongation step at 72°C.

A 5 sec picture was taken through the side of the optical block in the forbidden angle at cycle 0, 10, 15, 20, 25, 30, 35 and 40 to detect the micro-array in the detection chamber when this chamber was free of liquid.

Analysis of these raw pictures was made with Maxim DL software (Diffraction Limited, Ottawa, Canada) and quantification of raw signal intensities for three spots were performed: 1 GUT capture probe spot, local background of this spot and 1 spot of detection control. The raw signal intensities were plotted against the cycle number and results were provided in figure 1.

As a control, 100 pL of this PCR mix were processed in a PCR tube in a Mastercycler (Eppendorf, Hamburg, Germany) with the program: 5 min at 95°C followed by 40 cycles with 30 sec denaturation step at 95°C, 90 sec for annealing step at 58°C and 30 sec for elongation step at 72°C, followed by 10 min at 72°C before going down to a temperature of 4°C.

After 40 PCR cycles, 1 µL of the PCR solution performed in tube and 1 pL of PCR made in flow chip device were loaded onto Bioanalyser DNA 2000 gel cartridge (Agilent, Santa Clara Ca, USA). The intensities of the band corresponding to GUT amplicon were measured and transformed into amplicon concentration. The data gave a value of 34 nM for the flow chip device and 32 nM for the PCR tube showing that the PCR yields were comparable.

### Example 2: real-time multiplex PCR detected onto a micro-array into a flow chip device

A flow chip device was prepared as provided in example 1.

500 pL of a PCR mix was prepared in the buffer of example 1 with a mix of 16 couples of primers (primer mix 3.4) at 50 nM/75 nM (for labeled primer) containing the couple a primer for amplification of S. aureus (5'Oyster-TCAGTCTTACCTGCTCGATTC-3' (SEQ ID NO. 5) and 5'-TGCACGTCTAATACCACTCT-3' (SEQ ID NO. 6), Supersalt Taq polymerase 1U/50 gL, dNTP mix 200 pM (for dATP, dCTP, dGTP), 100 µM of dTTP and 300 pM of dUTP and 1 million copies of genomic S. aureus target DNA.

400 µL of the PCR mix were incubated at 99°C for 5 min before entering a flow chip device inside the flow instrument and were processed with the program: 48 cycles of 1 min 10 sec with a denaturation step at 99°C, annealing step at 58°C and elongation step at 72°C.

The flow device had a total volume of 2 mL and contained a detection chamber in the annealing zone. This detection chamber holds a micro-array (like in example 1) of capture probes with a capture probe specific for the target S. aureus amplicons, comprising a capture portion and a spacer portion (underlined).

S. aureus capture probe (SEQ ID NO. 7):

5'NH2-TTATTCACAACATTTCGATTTTTGCAACTACTTCAGTTCACTCCAAATTA
TGTTAAGTTATGTGGTGGAATATTCGTTGCCATACCTACCGC-3'

A 5 sec picture of this detection chamber was taken in forbidden angle at cycle 0, 3, 6, 9, 12, 15, 18, 21, 24, 27, 30, 33, 36, 39, 42, 45 and 48 cycle without liquid in the detection chamber. Analysis of these raw pictures were made with Maxim DL software and quantification of raw signal intensities for 1 spot of S. aureus capture probe minus raw local background of this spot were plotted against the cycle number. Results were provided in figure 2.

As a control, 100 pL of the PCR solution were processed in a PCR tube in a Mastercycler (Eppendorf, Hamburg, Germany) with the program: 5 min at 95°C followed by 48 cycles with 30 sec denaturation step at 95°C, 90 sec for annealing step at 58°C and 30 sec for elongation step at 72°C, followed by 10 min at 72°C before going down to a temperature of 4°C.

After 48 PCR cycles, 1 µL of PCR in tube and 1 µL of PCR made in flow chip device have been loaded onto Bioanalyser DNA 2000 gel cartridge. The intensities of the band corresponding to S. aureus amplicon have been measured and transformed into amplicon concentration (nM). The data gave a value of 10 nM for the flow chip device and 41 nM for the tube.

### Example 3: Signal evolution and treatment of the Real-time PCR signal on micro-array in a flow chip device

The assay was performed as in example 1 with the difference that the simplex PCR was performed using a different primer pair for P35 amplification:
- 5'Cy5-CGTCTTCAAAGCAAGTGGATTG-3' (SEQ ID NO. 8) and
- 5'-TCTTGCGAAGGATAGTGGGATT-3' (SEQ ID NO. 9).

The detection chamber holds a micro-array (like in example 1) of capture probes with a capture probe specific for the target P35 amplicons, comprising a capture portion and a spacer portion (underlined):
P35 capture probe (SEQ ID NO. 10):

5’NH2-
ATAAAAAAGTGGGTCTTAGAAATAAATTTCGAAGTGCAATAATTATTATTCACAACA
TTTCGATTTTTGCAACTACTTCAGTTCACTCCAAATTAGTCATCCCTTACGTCAGTGG
AGATAT-3’

The PCR was performed on 1 million copies of P35 target DNA and detection of the PCR product hybridized onto its specific capture molecule on the micro-array was performed at cycles 0, 3, 6, 9, 12, 18, 21, 24, 27, 30, 33, 36, 39 and 40 within the forbidden angle without the fluorescent liquid being present in the detection chamber. A graph showing the signals of the bound target P35 obtained at the different PCR cycles was shown in figure 3a. The graph shows the raw values and standard deviation of 1 spot (n°2) and the raw values and standard deviation for its local background.

Figure 3b shows the values of signal and local background of the same spot as well as the standard deviation on signal of spot and on local background when the different images at each cycle were corrected by subtraction of the image of the cycle 3 and addition of an offset of 1500 (using "pixel math" function in Maxim DL5 software). The subtraction was made pixel by pixel.

### Example 4: design of integrated flow chip device

The flow chip device was a disposable plastic cartridge presented in figure 4. It had the dimension of a credit card (80 mm x 50 mm x 2 mm) and comprised a flow channel (channel section: 500 gm x 500 gm) inside 4 different temperature regions (Region 1: channel of 20 cm long; Region 2: channel of 40 cm long; Region 3: 15 cm long and region 4: 20 cm long) and an interconnected detection chamber in region 2. The liquid movement was in a closed circuit after having connected the pump port of the channel in Region 1 and the pump port of the channel in Region 4 to an external pump being part of the Flow instrument through flexible pump tubing (#049.EF0A.051, Watson-Marlow, Stockholm, Sweden). The detection chamber has a hexagonal shape and was 10 mm (width) x 20 mm (length) x 100 gm (height). The detection chamber was closed on the upper side by a black plastic enclosure 1.5 mm thick and on the bottom side by an optical bloc 3.5 mm thick. The detection chamber was designed to hold about 15 µL of solution. The optical block was in Zeonex of optical grade. The surface was perfectly smooth and flat with scratches and dusts being avoided.

The introduction of a solution into the flow device was performed through one of the pump ports of Region 1 or 4. Once the liquid was into the flow chip device, it was closed and the pump tubing was introduced into the peristaltic pump (#Serie 100, Watson-Marlow, Stockholm, Sweden) so the liquid could flow in the closed loop flow chip device. The total volume capacity of the flow chip device including the flow channel and the detection chamber was about 240 pL. The flow chip device was designed to fit inside the Flow PCR instrument described in example 5 and schematically provided in figures 5 and 6.

### Example 5: design of integrated Flow PCR instrument

The instrument (F-RAP instrument) allowed both thermal cycling for amplification and liquid flow in the flow chip device as described in example 4 and shown in figures 5 and 6. It was composed of 4 heated blocks located both above and below the cartridge so that a total of 8 physically separated heating elements were present in the instrument. Each of the 4 temperature regions of the cartridge was sandwiched between a pair of heating aluminium blocks, having a thermal pad element on their surfaces to improve conductivity between the aluminium blocks and the plastic cartridge. Heat sources were heating resistances placed inside the aluminium blocks and connected to a voltage source.

To avoid excessive heat gradients, each heating element was separated from each other by the plastic structure and a thin air layer. Aluminium blocks were intentionally made slightly smaller that the polyoxymethylene (POM) pockets in which they were placed to reduce conductivity between the heating blocks and their surrounding holders.

Each heating block was thermally regulated by a thermocouple placed inside the aluminium elements. An external PID regulating electronic device (OMRON) sent power to the heating resistance at regular interval to maintain the desired temperature.

Air pulse radiators were connected to each heating aluminium blocks to allow them to go down in temperature in a reasonable time when needed.

A slight pressure was used to squeeze the cartridge between the heating elements and provide good thermal contact.

The instrument contained an optical detection system comprised of a high intensity laser source (nLight Pearl™ 2W) plus a lens, emission filter and a sensitive CCD camera (Kodak KAF 6303) to take pictures of the micro-array while illuminated by the laser.

One opening was made in the cartridge holder on the lower side so that the laser light source reached the micro-array for illumination from below. Another opening was made at 76° from the illumination axis to allow the emitted light coming from the micro-array to be detected by the CCD camera within the so called forbidden angle.

In order to move the fluid within the plastic cartridge, a short piece of tubing was connected to the cartridge and stick out of the heated system. This tubing was placed inside the small peristaltic pump (Marlow) that was controlled between 1 and 500 rpm (Faulhaber electric servo DC motor).

A thermocouple probe (sensor) was connected to the external surface of the tubing in order to detect the fluid passing. The liquid carried a higher thermal energy than the gas phase and hence showed a spike in the temperature profile when passing at the level of that probe. This temperature change was recorded and the timing of the liquid in the detection chamber was calculated. The sensor also allowed counting the number of cycles.

### Example 6: Amplicon accumulation on capture probes alone the cycles in an integrated flow chip device

In this example, the flow chip device was a disposable plastic cartridge provided in figure 4 and example 4. It has the dimension of a credit card (80 mm x 50 mm x 2 mm) and comprises a flow channel (channel section: 500 µm x 500 µm) inside 4 different temperature regions. The flow-chip device was designed to fit in the F-RAP instrument as provided in figures 5 and 6 and example 5.

The instrument (F-RAP) allows both thermal cycling for amplification and liquid flow in the flow device.

For this experiment, the optical block has been spotted with different capture probes: three of these capture probes were:
- Detection control: (SEQ ID NO. 1):

5'NH2-

TACCTACTACGCTACACGAACCTACAAGACAAGATAAAGACAGACTCATG-3'Cy5.

This labeled capture probe was spotted at a concentration of 500 nM.
- GUT capture probe: (SEQ ID NO. 11): 5’NH2- 
ATTCTTATATCTTTACCTTTTCATCTTAACTACTCTACCTCTCATTTATTGTGCGCCCC
AGCCCTCACGGCATGATG-3’
- A. baumanii capture probe: (SEQ ID NO. 12):

5'NH2-

TTATTCACAACATTTCGATTTTTGCAACTACTTCAGTTCACTCCAAATTA
TTAATTAACGGTGCTGCTGGTATTGCTGTAGGTATGGC-3’

The GUT and A. baumanii capture probes were spotted at a concentration of 12 µM and comprise a spacer portion (underlined).

A hybridization mix was prepared containing Hybridization Mastermix 2.0 (Eppendorf, Hamburg, Germany), 20 nM of GUT detection probe labeled with Oyster (5'Oyster-CATCATGCCGTGAGGGCTGGGGCGCACAGTCAAATTA-3', (SEQ ID NO. 13)), 1 nM of purified A. baumanii amplicons (generated with the primer: 5'Oyster-GCATTCACAACTTCTGTCATG-3', (SEQ ID NO. 14) and the primer: 5'-TACCGAAGTCCGTATGACTAAG-3', (SEQ ID NO. 15)). 50 µL of this hybridization mix was introduced into the flow cartridge, then the cartridge was closed by connecting the 2 pump ports to the pump tubing and introduced into the F-RAP instrument. The 4 lower heating blocks (temperature regions 1,2, 3 and 4 of the cartridge) were set respectively at 95°C, 58°C, 58°C and 72°C and the upper heating blocks (temperature regions l,2, 3 and 4 of the cartridge) were set respectively at 95°C, 58°C, 58°C and 72°C. The thermocouple probe was fixed on the pump tubing (just after the temperature region set at 72°C) and the flow of liquid recorded to count the number of cycles of hybridization. The pump speed was adjusted to 13 rpm (v200).

A picture of 5 sec was taken at cycle 0, 1, 2, 3, 4, 5, 6, 10 and 15 when the detection chamber was in gas phase.

Analysis of these raw pictures was made with Maxim DL software (Diffraction Limited, Ottawa, Canada) and quantification of raw signal intensities for 1 spot of GUT capture probe minus raw local background of this spot were plotted against the cycle number as well as raw signal intensities of 1 spot of A. baumanii capture probe minus raw local background signal of this spot. Results were provided in figure 8.

### Example 7: Real-time multiplex PCR detected onto a micro-array into an integrated flow chip device

In this example, the flow chip device was a disposable plastic cartridge provided in figure 4 and example 4. It has the dimension of a credit card (80 mm x 50 mm x 2 mm) and comprises a flow channel (channel section: 500 µm x 500 µm) inside 4 different temperature regions. The flow-chip device was designed to fit in the F-RAP instrument as provided in figures 5-6 and example 5.

The instrument (F-RAP) allows both thermal cycling for amplification and liquid flow in the flow device.

The flow device had a total volume of about 240 µL and contained a detection chamber in the annealing zone. This detection chamber holds a micro-array (like in example 1) of capture probes with a capture probe specific for the target H. influenzae amplicons, comprising a capture portion and a spacer portion (underlined):
- H. influenzae capture probe: (SEQ ID NO. 16):

5'NH2-

TTATTCACAACATTTCGATTTTTGCAACTACTTCAGTTCACTCCAAATTA
CTCAATGAGAAATATTGCTGATGGGTTTTGGATATCCTGAAGA -3'

100 µL of a PCR mix containing the PCR Buffer 1X (Taps 50 mM, Tris-HCL 95 mM, MgCl₂ 2 mM), Tween-20 0.1%, a mix of 29 couples of primers (primer mix 4.0) at 50 nM/75 nM (for labeled primer) containing the couple a primer for amplification of H. influenzae (5'Oyster-CGTGGCATTGCGAATTTCT-3' (SEQ ID NO. 17) and 5'-TACGGCGTTAAACGTCCTAAAG-3' (SEQ ID NO. 18), Supersalt Taq polymerase 1U/50 µL, dNTP mix 200 µM (for dATP, dCTP, dGTP), 100 µM of dTTP and 300 µM of dUTP and 5 10⁵ copies of genomic H. influenzae target DNA were prepared. The PCR buffer 1X also contained glutamate and dextran as provided in WO2009/086608.

100 µL of this PCR mix were incubated at 99°C for 5 min before being introduced into the flow cartridge, then the cartridge was closed by connecting the 2 pump ports to the pump tubing and introduced into the F-RAP instrument. The 4 lower heating blocks (temperature regions 1, 2, 3 and 4 of the cartridge) were set respectively at 95°C, 58°C, 58°C and 72°C and the upper heating blocks (temperature regions 1, 2, 3 and 4 of the cartridge) were set respectively at 95°C, 58°C, 58°C and 72°C. A thermocouple probe was fixed on the pump tubing (just after the temperature region set at 72°C) and the flow of liquid recorded to count the number of cycles of amplification. The pump speed was adjusted to 13 rpm (v200).

A picture of 5 sec was taken at cycle 2, 5, 10, 12, 14, 16, 18, 20, 21, 22, 23, 24, 25, 26, 27, 28, 29, 30, 31, 32, 33, 34, 35, 36, 37, 38, 39 and 40 when the detection chamber was in gas phase. The cycle numbers were obtained by determination of the number of spikes in the temperature profile when the solution was at the thermocouple probe level.

Analysis of these raw pictures was made with Maxim DL software (Diffraction Limited, Ottawa, Canada) and quantification of raw signal intensities of 1 spot of H. influenzae capture probe minus raw local background signal of this spot. Results were provided in figure 10a. The amplicon of H. influenzae is detected at cycle 26. The cycle numbers as counted by the thermocouple probe was provided in figure 10b.

### Example 8: Real-time multiplex PCR detected onto a micro-array into an integrated flow chip device using different volumes of PCR solution

The experiment was performed as described in example 7 with 5 10⁵ copies of genomic H. influenzae as target DNA.

PCR was performed in three flow chip devices which were filled with different volumes of PCR solution: either 50 µL, 150 µL or 200 pL. The PCR mix were incubated at 99°C for 5 min before being introduced into the flow cartridge, then the cartridge was closed by connecting the 2 pump ports to the pump tubing and introduced into the F-RAP instrument. The 4 lower heating blocks (temperature regions 1, 2, 3 and 4 of the cartridge) were set respectively at 95°C, 58°C, 58°C and 72°C and the upper heating blocks (temperature regions 1, 2, 3 and 4 of the cartridge) were set respectively at 95°C, 58°C, 58°C and 72°C. A thermocouple probe was fixed on the pump tubing (just after the temperature region set at 72°C) and the flow of liquid recorded to count the number of cycles of amplification. The pump speed was adjusted to 13 rpm (v200).

For the device having 50 µl of solution, a picture of 5 sec was taken at cycle 1, 2, 3, 4, 5, 6, 7, 8, 10, 12, 14, 16, 18, 20, 21, 22, 23, 24, 25, 26, 27, 28, 29, 30, 31, 32, 33, 34, 35, 36, 37, 38, 39 and 40 when the detection chamber was in gas phase.

For the device having 150 µL solution, a picture of 5 sec was taken at cycle l, 2, 3, 4, 5, 7, 10, 12, 14, 16, 18, 20, 21, 22, 23, 24, 25, 26, 27, 28, 29, 30, 31, 32, 33, 34, 35, 36, 37, 38, 39 and 40 when the detection chamber was in gas phase.

For the device having 200 µul of solution, a picture of 5 sec was taken at cycle 3, 4, 5, 6, 7, 10, 13, 16, 17, 18, 19, 20, 21, 22, 23, 24, 25, 26, 27, 28, 29, 30, 31, 32, 33, 34, 35, 36, 37, 38, 39 and 40 when the detection chamber was in liquid phase.

For the volumes of 50 µL and 150 µL, the cycle numbers were obtained by determination of the number of spikes in the temperature profile when the solution was at the thermocouple probe level. The spike is given by the passage of the solution front at the sensor location and one spike corresponds to one cycle of amplification. For the volume of 200 pL, the cycle duration was calculated to be 100 sec according to the parameters of the system (the flow channel section, its length inside the different temperature regions of the cartridge, the detection chamber volume and the flow rate of the solution).

Analysis of these raw pictures was made with Maxim DL software (Diffraction Limited, Ottawa, Canada) and quantification of raw signal intensities of 1 spot of H. influenzae capture probe minus raw local background signal of this spot. Results were provided in figure 1 l. The volume of the PCR solution had a strong impact on the detection cycle. Unexpectedly, greater was the PCR solution volume, earlier is the amplicon detected. The highest volume of 200 pL gave the lowest detection cycle (cycle 21), then the volume of 150 pL (cycle 22) and 50 µl (cycle 34).

## Claims

1. A method for performing real-time amplification and detection of target polynucleotide molecule(s) present in a sample, comprising the steps of:
a) providing a flow chip device comprising:
- a flow channel having a section comprised between 0.01 and 10 _{MM}² and a volume Vl, wherein the flow channel is configured such that a solution introduced into the flow channel is cycled through the flow channel,
- a detection chamber in fluid communication with the flow channel, said detection chamber having an optically transparent solid support and a micro-array comprising more than 4 capture molecules being immobilized in localized areas of the surface of said transparent solid support, wherein said chamber has a height lower than 1 mm and a volume V2, and wherein the ratio V2/V is between 0.001 and 0.5,
- at least 2 and preferably 3 different temperature regions at which temperature is regulated, each temperature region being located at a different location of the flow channel, wherein one temperature region comprises the detection chamber and has a temperature allowing the hybridization of the target polynucleotide molecules to the capture molecules,
b) introducing a solution having a volume V3 and containing target polynucleotide molecules into the flow channel and reagents for polynucleotide molecule amplification, wherein the ratio V3/(Vl+V2) is higher than 0.02 and lower than l,
c) submitting the solution to at least 5 amplification cycles to obtain labeled target polynucleotide molecules, wherein one amplification cycle is obtained by cycling the solution through the flow channel and the same detection chamber between the different temperature regions and wherein an amplification cycle is performed in less than 3 min,
d) measuring a fluorescent signal at different timings of the amplification from the hybridized target polynucleotide molecules in at least 3 and preferably in at least 5 different amplification cycles by detecting the fluorescence emitted from the localized areas of the surface having hybridized target polynucleotide measured after excitation of the fluorochrome by a light beam,
e) analyzing the signal values obtained from the localized area in order to detect and/or to quantify the target polynucleotide molecule(s) present in the sample.

2. The method of claim 1, wherein the amplification cycle is performed in less than 2 min and preferably in less than 1 min.

3. The method of any one of the preceding claims, wherein the detection chamber has a shape selected from elliptic, oval, rhomboidal, hexagonal, octagonal, diamond.

4. The method of any one of the preceding claims, wherein the volumes Vl+V2+V3 comprises two phases being a gas phase and a liquid phase.

5. The method of any one of the preceding claims, wherein the ratio V2/V is between 0.01 and 0.1.

6. The method of any one of the preceding claims, wherein the ratio V3/(Vl+V2) is higher than 0.5, preferably higher than 0.75, even higher than 0.9 and lower than 1.

7. The method of any one of the preceding claims, wherein the different timings of the amplification for measuring the fluorescent signal correspond to amplification cycles.

8. The method of claim 7, wherein the amplification cycles are determined by data analysis of a flow chip sensor.

9. The method of claim 7, wherein the amplification cycles are calculated from V 1+V2 of the flow chip device features and flow rate.

10. The method of any one of the preceding claims, wherein the amplification is obtained by PCR comprising denaturation, annealing and elongation steps.

11. The method of any one of the preceding claims, wherein the labeled target polynucleotide molecules are fluorescently labeled by incorporation of a fluorochrome labeled amplification precursor.

12. The method of any one of the preceding claims, wherein the detection of the fluorescence emitted from the localized areas of the surface having hybridized target polynucleotide is assayed through an optically transparent solid support bearing the immobilized capture molecules in an observation angle which is within the forbidden angle.

13. The method of any one of the preceding claims, wherein the capture molecule has a spacer of at least 6.8 nm long being preferably a sequence of at least 20 nucleotides and preferably more than 40 nucleotides long.

14. The method of any one of the preceding claims, wherein the micro-array comprises fluorescently labeled capture molecules which keep more than 50% of their fluorescence, preferably more than 80% and even better more than 90% at cycle 35 of the amplification as compared to cycle 1.

15. The method of any one of the preceding claims, wherein the measurement of a fluorescent signal from the hybridized target polynucleotide molecules is performed in a gas phase.

16. The method of any one of claims 1-14, wherein the measurement of a fluorescent signal from the hybridized target polynucleotide molecules is performed in the presence of the amplification solution containing the labeled target polynucleotide molecules.

17. The method of any one of the preceding claims, wherein the height of the liquid in the detection part of the detection chamber is preferably comprised between 10 and 250 µm and preferably between 50 and 150 um.

18. The method of any one of the preceding claims, wherein the location of the amplification solution in the flow channel and/or in the detection chamber is known by a time control of the liquid phase location in the flow channel.

19. The method of claim 18, wherein the liquid phase location is known from a measure of a signal obtained in at least one location of the flow channel, from a gas/liquid phase transition, said measure being obtained by temperature shift, fluorescence signal change, electric signal, luminescence or light absorbance change.

20. The method of any one of the preceding claims, wherein cumulative spot values are plotted along the amplification cycles to detect and/or to quantify the target polynucleotide molecule(s) present in the sample.

21. The method of any one of the preceding claims, wherein the quantification of the target polynucleotide molecule(s) present in the sample is obtained by comparing the number of amplification cycles necessary to reach a fixed value (CT) with the CT of a reference polynucleotide molecule.

22. The method of any one of the preceding claims, wherein the analysis the signal values is performed on a micro-array image having pixel values corrected by the pixel values of the image taken before the amplification or in one of the first ten amplification cycles.

23. A method of assay of a phase transition within a microfluidic device for follow up real-time PCR cycles comprising the steps of:
a) providing a flow chip device comprising:
- a flow channel having a section comprised between 0.01 and 10 _{MM}² and a volume Vl, wherein the flow channel is configured such that a solution introduced into the flow channel is cycled through the flow channel,
- a detection chamber in fluid communication with the flow channel, said detection chamber having an optically transparent solid support comprising at least one capture molecules being immobilized in localized areas of the surface of said transparent solid support, wherein said chamber has a height lower than 1 mm and a volume V2,
- at least 2 and preferably 3 different temperature regions at which temperature is regulated, each temperature region being located at a different location of the flow channel, wherein one temperature region comprises the detection chamber and has a temperature allowing the hybridization of the target polynucleotide molecules to the capture molecules,
b) a flow chip sensor for the detection of a phase transition of liquid/air and/or air/liquid,
c) introducing a solution having a volume V3 and containing target polynucleotide molecules into the flow channel and reagents for polynucleotide molecule amplification, wherein the ratio V3/(V1+V2) is higher than 0.02 and lower than l,
d) submitting the solution to at least 5 amplification cycles to obtain labeled target polynucleotide molecules, wherein one amplification cycle is obtained by cycling the solution through the flow channel and the same detection chamber between the different temperature regions and wherein an amplification cycle is performed in less than 3 min,
e) determining the amplification cycles by data analysis of a flow chip sensor,
f) measuring a fluorescent signal at different cycles of the amplification from the hybridized target polynucleotide molecules in at least 3 and preferably in at least 5 different amplification cycles by detecting the fluorescence emitted from the localized areas of the surface having hybridized target polynucleotide measured after excitation of the fluorochrome by a light beam,
g) analyzing the signal values obtained from the localized area along the amplification cycles in order to detect and/or to quantify the target polynucleotide molecule(s) present in the sample.

24. A Flow-chip device for performing real-time amplification and detection of target polynucleotide molecule(s) present in a sample, comprising:
- a flow channel disposed within a substrate, said flow channel having a section comprised between 0.01 and 10 mm² and a volume VI, wherein the flow channel is configured such that a solution introduced into the flow channel is cycled through the flow channel,
- a detection chamber connected to the flow channel, said detection chamber having fixed upon one of its surface a micro-array comprising more than 4 capture molecules being immobilized in localized areas of said surface, wherein said chamber has a height lower than 1 mm and a volume V2, and wherein the ratio V2/V1 is between 0.001 and 0.5,
- at least 2 and preferably 3 different temperature regions at which temperature is regulated, each temperature region being located at a different location of the flow channel, wherein one temperature region comprises the detection chamber and has a temperature allowing the hybridization of the target polynucleotide molecules to the capture molecules,
- an inlet in fluid communication with the flow channel via which the solution is introduced into the flow channel,
wherein the capture molecules are immobilized on the surface (Sl) of an optically transparent solid support having a refractive index higher than 1.3 and a thickness of at least 0.5 mm and preferably at least 3 mm, wherein said solid support has two surfaces (S2 and S3) inclined relative to the surface of the support on which the capture molecules are immobilized, one (S2) being optically transparent and used for collecting light emitted from the localized areas of capture molecules and inclined by an angle of between 90 and 60° compared tot the solid support surface and the other one (S3) opposite being black or covered with a colour being black or covered with a colour having an absorption corresponding to the wavelength of the emitted light.

25. The flow chip of claim 24, wherein the substrate comprises an opaque polymer.

26. An apparatus for performing real-time amplification and detection of target polynucleotide molecule(s) present in a sample, comprising:
a) a flow chip device comprising:
- a flow channel having a section comprised between 0.01 and 10 _{MM}² and a volume Vl, wherein the flow channel is configured such that a solution introduced into the flow channel is cycled through the flow channel,
- a detection chamber connected to the flow channel, said detection chamber having fixed upon one of its surface a micro-array comprising more than 4 capture molecules being immobilized in localized areas of said surface, wherein said chamber has a height lower than 1 mm and a volume V2, and wherein the ratio V2/V is between 0.001 and 0.5,
- at least 2 and preferably 3 different temperature regions at which temperature is regulated, each temperature region being located at a different location of the flow channel, wherein one temperature region comprises the detection chamber and has a temperature allowing the hybridization of the target polynucleotide molecules to the capture molecules;
b) a holder for the flow chip device;
c) a heating system in front of the at least 2 different temperature regions;
d) a temperature controller disposed to regulate temperature within the at least 2 different temperature regions;
e) optionally a flow chip sensor;
f) an illumination light source;
g) a system operatively disposed to transport fluid through the flow channel;
h) a detector for measuring a fluorescent signal from the hybridized target polynucleotide molecules, wherein the surface of emission for a localized area is comprised between about 0.1 mm2 and about 75mm², wherein the detection of the fluorescence emitted from the localized areas of the surface having hybridized target polynucleotide molecules is assayed through an optically transparent solid support bearing the immobilized capture molecules in an observation angle which is within the forbidden angle;
wherein the different parts are integrated into the same apparatus and wherein the position of the flow chip device is fixed compared to the detector.

27. The apparatus of claim 26, wherein the capture molecules are immobilized on the surface of an optically transparent solid support having a refractive index higher than 1.30 and a thickness of at least 0.5 mm and preferably at least 3 mm, wherein said solid support has two surfaces (S2 and S3) inclined relative to the surface (Sl) of the support on which the capture molecules are immobilized, one (S2) being optically transparent and used for collecting light emitted from the localized areas of capture molecules in the forbidden angle (θobin) and inclined by an angle of between 90 and 60° compared to the solid support surface and the other one (S3) opposite being black or covered with a colour being black or covered with a colour having an absorption corresponding to the wavelength of the emitted light and wherein the device is positioned onto the apparatus in order for the light emitted in the forbidden angle (θobin) trough the inclined surface S2 to reach the detector.

28. The apparatus according to any one of claims 26-27, wherein the flow chip sensor is a heat detector, a fluorescence detector or a light absorbance detector.

29. The apparatus according to any one of claims 26-28, further comprising:
- a storage system for storing the data of the different measurements,
- a controller repeating the steps of illumination, detection and storage,
- a data analysis of the flow chip sensor to determine the liquid position in the flow chip device,
- a program for processing the data in order to detect and/or quantify the amount of polynucleotide molecule present in the solution before the amplification.
